# EUROPEAN PATENT APPLICATION

(11) **EP 4 407 032 A1**
(43) Date of publication of application: **31.07.2024**
(21) Application number: 22872191.6
(22) Date of filing: 26.09.2022
(51) Int. Cl.: C12N 15/115, A61K 31/7115, A61P 35/00, A61P 19/02, A61P 29/00

(54) **APTAMER FOR DKK1 AND USE THEREOF**

(30) Priority: 26.09.2021 CN 202111127857
(71) Applicant: Aptacure Therapeutics Limited, New Territories, Hong Kong 999077 (HK)
(72) Inventor: YU, Yuanyuan, Hong Kong, 999077 (CN); HE, Yixin, Hong Kong, 999077 (CN); CHU, Hangyin, Hong Kong, 999077 (CN); ZHANG, Ge, Hong Kong (CN)
(74) Representative: Nederlandsch Octrooibureau
(86) International application number: PCT/CN2022/121254
(87) International publication number: WO 2023/046147

(57) **Abstract**

The present invention relates to the field of biomedicine. Specifically, the present invention relates to an aptamer against DKK1 and uses thereof, in particular to uses in the treatment of DKK1-related diseases such as DKK1-related cancer.

## Description

### Field of the Invention

The present invention relates to the field of biomedicine. Specifically, the present invention relates to an aptamer against DKK1 and uses thereof, in particular to uses in the treatment of DKK1-related diseases such as DKK1-related cancer.

### Background

Dickkopf-1 (DKK1) is a member of the dickkopf protein family and has been proved to be a negative regulator of Wnt signaling, and it plays a core role in bone development and formation. By binding to LRP5 (LRP6) and kremen protein, DKK1 prevents LRP5 or LRP6 from binding to members of Wnt pathway, thereby preventing the Wnt-mediated signaling, and inhibiting the bone formation.

In clinical researches, an increased level of the Wnt antagonist DKK1 has been detected in the serum or tumors of the patients with a series of cancers, and this is often associated with poor prognosis. In addition, DKK1 promotes the immunosuppressive tumor microenvironment and induces the tumors to evade immune surveillance by inhibiting T cells. A humanized monoclonal therapeutic antibody against DKK1 named DKN-01, combined with chemotherapy, has been used in Phase-II trials to treat an esophageal gastric cancer and other types of cancers. Therefore, DKK1 may be a promising target for tumor immunotherapy. However, there are several major concerns for therapeutic antibodies, including high immunogenicity (Padhi, Jang et al. 2011, Padhi, Allison et al. 2014), expensive and laborious to produce (Baker 2015, Bradbury and Pluckthun 2015, Groff, Brown et al. 2015), unstable which requires a continuous cold chain for transportation and storage (Jayasena 1999). Therefore, an alternative anti-DKK1 agent with no immunogenicity, easy production, low cost and high stability is desirable for bone anabolic therapy.

Aptamers have attracted widespread attention due to their high binding affinity and specificity and low immunogenicity. Aptamers are short single-stranded oligonucleotides which bind to their targets through conformational complementarity (Ellington and Szostak 1990, Tuerk and Gold 1990). Compared to therapeutic antibodies, aptamers possess similar affinity and specificity, but have some important advantages. For immunogenicity, aptamers are not recognized by the immune system as foreign and do not stimulate a negative immune response because of the small size (Keefe, Pai et al. 2010). For production and cost, aptamers are identified *in vitro* under various selection conditions and can be easily synthesized by chemical methods, so production is less expensive and less risky (Banerjee 2010). For stability, aptamers have an indefinite shelf life as they are temperature resistant and can tolerate transportation without any special requirements for cooling, eliminating the need for a continuous cold chain (Jayasena 1999). Pegaptanib, an aptamer against vascular endothelial growth factor (VEGF) for the treatment of age-related macular degeneration has been successfully used in clinic (Jellinek, Green et al. 1994, Ruckman, Green et al. 1998, Ng and Adamis 2006, Que-Gewirth and Sullenger 2007).

Therefore, it is desired to develop aptamers against DKK1 to replace monoclonal antibodies for the treatment of DKK1-related cancers.

### Summary of the Invention

In one aspect, the present invention provides an aptamer against DKK1, wherein the aptamer
i) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 1-20; or
ii) comprises at least 30, at least 35, at least 40, at least 45, at least 50 or more contiguous nucleotides within any one of SEQ ID NOs: 1-20; or
iii) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 45-64; or
iv) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 87-105,
wherein the aptamer specifically binds to DKK1.

In some embodiments, the aptamer comprises a sequence of any one of SEQ ID NOs: 1-64 and 87-105.

In some embodiments, the aptamer comprises a nucleotide sequence shown in SEQ ID NO: 5, 20, 35-38, 49, or 64, preferably a nucleotide sequence shown in SEQ ID NO: 37. In some embodiments, the aptamer comprises one or more modifications that confer enhanced nuclease resistance to the aptamer and/or enhance the *in vivo* half-life of the aptamer.

In some embodiments, the aptamer comprises 2'-methoxy (2'-OMe) modification and 3' inverted deoxythymidine (3' idT) modification.

In some embodiments, the aptamer has a Kd to DKK1 of less than 350 nM, preferably less than 150 nM, preferably less than 100 nM, preferably less than 50 nM, preferably less than 30 nM, preferably less than 20 nM, preferably less than 10 nM, preferably less than 5 nM, preferably less than 1 nM or less.

In some embodiments, the aptamer is capable of inhibiting the biological activity of DKK1. In some embodiments, the aptamer can block an antagonistic effect of DKK1 in cell-based Wnt signaling assay. In some embodiments, the aptamer has an IC50 value of less than 2000Nm, preferably less than 1500nM, preferably less than 1000nM, preferably less than 800nM or less for inhibiting the biological activity of DKK1, for example, inhibiting the antagonistic effect of DKK1 on Wnt signaling pathway.

In another aspect, the present invention provides a method for treating DKK1-related diseases, the method comprises administering a therapeutic effective amount of the aptamer against DKK1 of the present invention to a subject in need thereof, and the subject is, for example, a human.

In some embodiments, the DKK1-related disease is a DKK1-related cancer, such as myeloma (e.g., multiple myeloma with osteolytic lesions, hilar cholangiocarcinoma multiple myeloma), breast cancer, colon cancer, melanoma, hepatocellular cancer, epithelial cancer, esophageal cancer, gastric cancer, gastroesophageal cancer, hilar cholangiocarcinoma, brain cancer, lung cancer, prostate cancer, or pancreatic cancer, as well as any metastases thereof.

In some embodiments, the DKK1 related disease is selected from osteoporosis, osteopenia, osteomalacia, osteogenesis imperfecta (OI), ischemic osteonecrosis, rheumatoid arthritis, fracture, osteoarthritis, and myeloma.

In another aspect, the present invention provides a pharmaceutical composition comprising at least one aptamer against DKK1 of the present invention and a pharmaceutically acceptable carrier or excipient.

In another aspect, the present invention provides use of the aptamer against DKK1 of the present invention or the pharmaceutical composition of the present invention in preparation of a medicament for treating DKK1-related diseases.

In some embodiments, the DKK1 related disease is a DKK1-related cancer, such as myeloma (e.g., multiple myeloma with osteolytic lesions, hilar cholangiocarcinoma multiple myeloma), breast cancer, colon cancer, melanoma, hepatocellular cancer, epithelial cancer, esophageal cancer, gastric cancer, gastroesophageal cancer, hilar cholangiocarcinoma, brain cancer, lung cancer, prostate cancer, or pancreatic cancer, as well as any metastases thereof.

In some embodiments, the DKK1-related disease is selected from osteoporosis, osteopenia, osteomalacia, OI, ischemic osteonecrosis, rheumatoid arthritis, fracture, osteoarthritis, and myeloma.

### Brief Description of the Drawings

Fig. 1 shows the tested binding ability of Ni-NTA magnetic agarose beads to a DKK1 protein. Two experiments were performed to detect the binding ability of the Ni-NTA magnetic agarose beads to the DKK1 protein. In sodium dodecyl sulfate polyacrylamide gel electrophoresis (SDS-PAGE) under a reducing condition, the apparent molecular weight of DKK1 is about 40 KDa. This condition (0.6 nmol DKK1 + 6 µl 5% beads) shows the highest binding ability.
Fig.2 shows agarose gel (3%) electrophoretic analysis of polymerase chain reaction (PCR) products of aptamer pools screened from round 1 to round 20. The first lane is a DNA ladder. DNA bands exist approximately at 80 bp.
Fig. 3 shows evolutionary tree and alignment of aptamer candidates. Twenty aptamer sequences are divided into two groups. The first group contains 8 guanine-rich sequences, known as a Grich group; and the second group includes twelve sequences belonging to other nucleotides, known as a non-G rich group.
Fig. 4 shows specificity of representative aptamer candidates. An enzyme-linked oligonucleotide assay (ELONA) is used to determine the specificity of 1 µM of each aptamer/RS against DKK1 and detect absorbance at 450 nm. **** represents P<0.0001; ** represents P<0.005; and * represents P<0.05.
Fig. 5 shows affinity of the representative aptamer candidates. The affinity against DKK-1 is detected with specific concentration of each aptamer and measured the absorbance at 450 nm.
Fig. 6 shows inhibition effects of the representative aptamer candidates. Compared with a control group, aptdkk-5, 19, and 20 show the higher ratios, indicating that they may relieve the inhibition on the Wnt signaling by DKK1.
Fig. 7 shows inhibitory efficacy of the representative aptamer candidates. aptdkk-5 has the relatively high inhibitory efficacy with IC₅₀=754 ± 29 nM, while aptdkk-20 has the relatively low inhibitory efficacy with IC₅₀=1504 ± 39 nM. Data of aptdkk-19 does not conform to a fitting curve.
Fig. 8 shows specificity of the truncated aptdkk5. The specificity against DKK-1 is detected with a) 100 nM and b) 1 nM of each truncated aptdkk5, and the absorbance at 450 nm is compared with the absorbance of the original aptdkk-5. * represents P<0.05; and NS represents no significant difference.
Fig. 9 shows affinity of the truncated aptdkk5. The binding affinity to DKK1 is detected with the gradient concentration of each aptamer and the absorbance at 450 nm is detected.
Fig. 10 shows specificity of Aptdkk5s mutants. The specificity of 1 µM of each aptamer to DKK-1 is detected by ELONA, and the absorbance at 450 nm is detected. **** represents P<0.0001; ** represents P<0.005; and * represents P<0.05.
Fig. 11 shows expression levels of DKK1 and defective DKK1 in HEK293T cells measured by SDS-PAGE gel electrophoresis. S4, pCS2-DKK1-His-flag; S3, pCS2-DKK1- Δ C-His-flag; and S2, pCS2-DKK1- Δ L2&C-His-flag.
Fig. 12 shows binding ability of full-length and truncated DKK1, and a fluorescence image of aptdkk5s binding to DKK1 mutants. S4, pCS2-DKK1-His-flag; S3, pCS2-DKK1-ΔC-His-flag; S2, pCS2-DKK1-ΔL2&C-His-flag; positive Ctrl, pure protein DKK1 His6; and negative Ctrl, beads only.
Fig. 13 shows specificity of modified aptdkk5s. The specificity of 1 µM of each aptamer to DKK-1 is detected by ELONA, and the absorbance at 450 nm is detected.
Fig. 14 shows affinity of modified aptddk5s. The affinity of a certain concentration of each aptamer to DKK-1 is detected by ELONA, and the absorbance at 450 nm is detected.
Fig. 15 shows evaluation of serum stability of modified aptdkk5s compared to unmodified aptdkk5s. All aptamers are treated with 10% and 100% fetal bovine serum from 0 to 72 hours respectively.
Fig. 16 shows inhibitory efficacy of aptdkk5-OM-indT. There are three copies of each group analysis.
Fig. 17 shows a sequence enrichment trend curve. The degree of enrichment of DNA pools in each round is calculated by dividing the type of the aptamer in each round by the number of sequencing reads. Saturation enrichment is believed to occur in round 24.
Fig. 18 shows agarose gel (3%) electrophoresis analysis of PCR products of the aptamer pools selected from round 21 to round 30. The front lanes are DNA ladders. DNA bands are approximately at 80 bp.
Fig. 19 shows specificity of the representative aptamer candidates in round 30. The specificity of 1 µM of each aptamer/RS against DKK1 is detected by ELONA, and the absorbance at 450 nm is detected.
Fig. 20 shows a binding curve of the representative aptamer candidates. The affinity for DKK-1 is detected with a certain concentration of each aptamer by biolayer interferometry (BLI).

### Detailed Description of the Invention

Unless indicated or defined otherwise, all terms used have their usual meaning in the art, which will be clear to the skilled person. Reference is for example made to the standard handbooks, such as Sambrook et al, "Molecular Cloning: A Laboratory Manual"; Lewin, "Genes IV"; and Roitt et al., "Immunology" (8th Ed.), as well as to the general background art cited herein. Furthermore, unless indicated otherwise, all methods, steps, techniques and manipulations that are not specifically described in detail can be performed and have been performed in a manner known per se, as will be clear to the skilled person. Reference is for example again made to the standard handbooks, to the general background art referred to above and to the further references cited therein.

### Definitions

As used herein, the term "nucleotide" refers to a ribonucleotide or a deoxyribonucleotide, or a modified form thereof, as well as an analog thereof. Nucleotides include species that include purines (e.g., adenine, hypoxanthine, guanine, and their derivatives and analogs) as well as pyrimidines (e.g., cytosine, uracil, thymine, and their derivatives and analogs). As used herein, "nucleic acid," "oligonucleotide," and "polynucleotide" are used interchangeably to refer to a polymer of nucleotides and include DNA, RNA, DNA/RNA hybrids and modifications of these kinds of nucleic acids, oligonucleotides and polynucleotides, wherein the attachment of various entities or moieties to the nucleotide units at any position are included. The terms "polynucleotide," "oligonucleotide," and "nucleic acid" include double- or single- stranded molecules. Nucleic acid, oligonucleotide, and polynucleotide are broader terms than the term aptamer and, thus, the terms nucleic acid, oligonucleotide, and polynucleotide include aptamers but are not limited to aptamers.

As used herein, "aptamer" refers to a non-naturally occurring nucleic acid that has a desirable action on a target molecule. A desirable action includes, but is not limited to, binding of the target, catalytically changing the target, reacting with the target in a way that modifies or alters the target or the functional activity of the target, covalently attaching to the target, and facilitating the reaction between the target and another molecule. In one embodiment, the action is specific binding affinity for a target molecule (such as, DKK1), such target molecule being a three dimensional chemical structure other than a polynucleotide that binds to the aptamer through a mechanism which is independent of Watson/Crick base pairing or triple helix formation, wherein the aptamer is not a nucleic acid having the known physiological function of being bound by the target molecule. In this context, the "specific binding" of an aptamer for its target (such as, DKK1) means that the aptamer binds to its target generally with a much higher degree of affinity than it binds to other, non-target, components in a mixture or sample.

Sequence "identity" has a well-known meaning and the percentage of sequence identity between two nucleic acid or polypeptide molecules or regions can be calculated using published techniques. Sequence identity can be measured along the full length of a polynucleotide or polypeptide or along a region of the molecule. (See, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G, eds., Humana Press, New Jersey, 1994; Sequence Analysis in Molecular Biology, von Heinje, G, Academic Press, 1987; and Sequence Analysis Primer, Gribskov, M. and Devereux, J., eds., M Stockton Press, New York, 1991). While there exist a number of methods to measure identity between two polynucleotide or polypeptides, the term "identity" is well known to skilled artisans (Carrillo, H. & Lipman, D., SIAM J Applied Math 48:1073 (1988)). One example of an algorithm that is suitable for determining percent sequence identity is the algorithm used in the basic local alignment search tool (hereinafter "BLAST"), see, e.g. Altschul et al, J. Mol. Biol. 215:403-410, 1990 and Altschul et al, Nucleic Acids Res., 15:3389-3402, 1997. Software for performing BLAST analyses is publicly available through the National Center for Biotechnology Information (hereinafter "NCBI"). The default parameters used in determining sequence identity using the software available from NCBI, e.g., BLASTN (for nucleotide sequences) are described in McGinnis et al, Nucleic Acids Res., 32:W20-W25, 2004.

### Aptamer against DKK1

Based on the protein-SELEX technology, the present inventors used DKK1 as the target protein for positive screening and unrelated proteins for negative screening, and finally selected aptamers that specifically bind to DKK1 with high affinity. The DKK1 described herein is preferably human DKK1, for example, the DKK1 whose amino acid sequence is shown in SEQ ID NO:73.

### Exemplary human DKK1 amino acid sequence:

MMALGAAGATRVFVAMVAAALGGHPLLGVSATLNSVLNSNAIKNLPPPLGGAA
GHPGSAVSAAPGILYPGGNKYQTIDNYQPYPCAEDEECGTDEYCASPTRGGDAG
VQICLACRKRRKRCMRHAMCCPGNYCKNGICVSSDQNHFRGEIEETITESFGND
HSTLDGYSRRTTLSSKMYHTKGQEGSVCLRSSDCASGLCCARHFWSKICKPVLK
EGQVCTKHRRKGSHGLEIFQRCYCGEGLSCRIQKDHHQASNSSRLHTCQRH
(SEQ ID NO: 73).

Therefore, in one aspect, the present invention provides an aptamer against DKK1. In some embodiments, the aptamer specifically binds to DKK1.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 1-20. In some embodiments, the aptamer consists of a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 1-20. In some embodiments, the aptamer comprises at least 10, at least 15, at least 20, at least 25, at least 30, at least 35, at least 40, at least 45, at least 50, at least 55, at least 60, at least 65 or more contiguous nucleotides within any one of SEQ ID NOs: 1-20. In some embodiments, the aptamer consists of at least 10, at least 15, at least 20, at least 25, at least 30, at least 35, at least 40, at least 45, at least 50, or more contiguous nucleotides within any one of SEQ ID NOs: 1-20.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 5. In some embodiments, the aptamer consists of a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 5. In some embodiments, the aptamer comprises at least 10, at least 15, at least 20, at least 25, at least 30, at least 35, at least 40, at least 45, at least 50, at least 55, at least 60, at least 65 or more contiguous nucleotides within SEQ ID NO: 5. In some embodiments, the aptamer consists of at least 10, at least 15, at least 20, at least 25, at least 30, at least 35, at least 40, at least 45, at least 50, or more contiguous nucleotides within SEQ ID NO: 5.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 19. In some embodiments, the aptamer consists of a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 19. In some embodiments, the aptamer comprises at least 10, at least 15, at least 20, at least 25, at least 30, at least 35, at least 40, at least 45, at least 50, at least 55, at least 60, at least 65 or more contiguous nucleotides within SEQ ID NO: 19. In some embodiments, the aptamer consists of at least 10, at least 15, at least 20, at least 25, at least 30, at least 35, at least 40, at least 45, at least 50, or more contiguous nucleotides within SEQ ID NO: 19.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 20. In some embodiments, the aptamer consists of a nucleotide sequence having at least about 90%, at least about 91%, at least about 92%, at least about 93%, at least about 94%, at least about 95%, at least about 96%, at least about 97%, at least about 98%, or at least about 99% identity with SEQ ID NO: 20. In some embodiments, the aptamer comprises at least 10, at least 15, at least 20, at least 25, at least 30, at least 35, at least 40, at least 45, at least 50, at least 55, at least 60, at least 65 or more contiguous nucleotides within SEQ ID NO: 20. In some embodiments, the aptamer consists of at least 10, at least 15, at least 20, at least 25, at least 30, at least 35, at least 40, at least 45, at least 50, or more contiguous nucleotides within SEQ ID NO: 20.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 45-64. In some embodiments, the aptamer comprises a nucleotide sequence of any one of SEQ ID NOs: 45-64 or consists of a nucleotide sequence of any one of SEQ ID NOs: 45-64. In some embodiments, the aptamer comprises a nucleotide sequence shown in SEQ ID NOs: 49, 63 or 64 or consists of a nucleotide sequence shown in SEQ ID NOs: 49, 63 or 64.

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 87-105. In some embodiments, the aptamer comprises a nucleotide sequence of any one of SEQ ID NOs: 87-105 or consists of a nucleotide sequence of any one of SEQ ID NOs: 87-105. In some embodiments, the aptamer comprises a nucleotide sequence of any one of SEQ ID NOs: 87-90, 93-96 and 98-105 or consists of a nucleotide sequence of any one of SEQ ID NOs: 87-90, 93-96 and 98-105.

In some embodiments, the aptamer comprises a nucleotide sequence of any one of SEQ ID NOs: 1-20. In some embodiments, the aptamer consists of a nucleotide sequence of any one of SEQ ID NOs: 1-20.

In some embodiments, the aptamer comprises a nucleotide sequence of any one of SEQ ID NOs: 21-44. In some embodiments, the aptamer consists of a nucleotide sequence of any one of SEQ ID NOs: 21-44.

In some embodiments, the aptamer comprises a nucleotide sequence of any one of SEQ ID NOs: 74-86. In some embodiments, the aptamer consists of a nucleotide sequence of any one of SEQ ID NOs: 74-86.

In some preferred embodiments, the aptamer comprises a nucleotide sequence of any one of SEQ ID NOs: 1-44, more preferably, the aptamer comprises a nucleotide sequence of any one of SEQ ID NOs: 5, 19, or 20-44, and more preferably, the aptamer comprises a nucleotide sequence of any one of SEQ ID NOs: 5, 20, or 35-38. In some preferred embodiments, the aptamer consists of a nucleotide sequence of any one of SEQ ID NOs: 1-44, more preferably, the aptamer consists of a nucleotide sequence of any one of SEQ ID NOs: 5, 19, or 20-44, and more preferably, the aptamer consists of a nucleotide sequence of any one of SEQ ID NOs: 5, 20, or 35-38. In some preferred embodiments, the aptamer comprises a nucleotide sequence shown in SEQ ID NO: 37 or consists of a nucleotide sequence shown in SEQ ID NO: 37.

In some embodiments, the aptamer of the present invention has a Kd (dissociation constant) to DKK1 of less than about 350 nM, preferably less than about 150 nM, preferably less than about 100 nM, preferably less than about 50 nM, preferably less than about 30 nM, preferably less than about 20 nM, preferably less than about 10 nM, preferably less than about 5 nM, preferably less than about 1 nM or smaller. In some embodiments, the aptamer of the present invention has a Kd (dissociation constant) to DKK1 of of about 0.01 nM - about 350 nM, preferably about 0.01 nM - about 150 nM, preferably about 0.01 nM - about 100 nM, preferably about 0.01 nM - about 50 nM, preferably about 0.01 nM - about 30 nM, preferably about 0.01 nM - about 20 nM, preferably about 0.01 nM - about 10 nM, preferably about 0.01 nM - about 5 nM, preferably about 0.01 nM - about 1 nM. The Kd is determined, for example, by enzyme-linked oligonucleotide assay (ELONA) or Bio-Layer Interferometry (BLI).

In some embodiments, the aptamer of the present invention inhibits the biological activity of DKK1. "Inhibition" means that the biological activity of DKK1 is reduced in the presence of the aptamer compared with the absence of the aptamer, for example, reduced by at least about 10%, at least about 20%, at least about 30%, at least about 40%, at least about 50%, at least about 60%, at least about 70%, at least about 80%, and even at least about 90%.

As used in this article, the term "biological activity" refers to an effect on one or more cellular or extracellular processes that may affect physiological or pathophysiological processes. The biological activity of DKK1 includes but not limited to antagonizing the Wnt signaling pathway.

In some embodiments, the aptamer of the present invention may inhibit the antagonistic effect of DKK1 on the Wnt signaling pathway. For example, the aptamer of the present invention may block the antagonistic effect of DKK1 in cell-based Wnt signaling assay.

In some embodiments, the aptamer of the present invention inhibits the biological activity of DKK1, such as inhibits the antagonistic effect of DKK1on the Wnt signaling pathway, with an IC₅₀ value of less than about 2000 nM, preferably less than about 1500 nM, preferably less than about 1000 nM, preferably less than about 800 nM, preferably less than about 400 nM, preferably less than about 200 nM or less. In some embodiments, the aptamer of the present invention inhibits the biological activity of DKK1, such as inhibits the antagonistic effect of DKK1on the Wnt signaling pathway, with an IC₅₀ value of about 200 nM - about 2000 nM, preferably about 200 nM - about 1500 nM, preferably about 200 nM - about 1000 nM, preferably about 200 nM - about 800 nM, or preferably about 200 nM - about 400 nM. In some embodiments, the IC₅₀ value is determined *in vitro* by a TCF-Wnt-induced luciferase activity assay in HEK293T cells.

In some embodiments, the aptamer of the present invention may further contain one or more modifications. For example, the modification is a modification that confers an enhanced nuclease resistance to the aptamer and/or enhances the *in vivo* half-life of the aptamer.

The modification includes, for example, 3' and 5' modifications, such as 3' and 5' capping. In some embodiments, the aptamer is capped with inverted deoxythymidine at the 3' end, that is, a 3' inverted deoxythymidine (3' idT) modification.

The modification may further include the substitution of one or more naturally occurring nucleotides with modified nucleotides. For example, the modified nucleotides include, but are not limited to, 2'-fluoro, 2'-methoxyethyl, 2'-methoxy and/or 2' allyloxy modified nucleotides (i.e., the 2'-position hydroxyl group of the ribose is substituted by fluorine, methoxyethyl, methoxy or allyloxy, etc.). The modified nucleotides may also include C-5 modified pyrimidines. The term "C-5 modified pyrimidine" refers to a pyrimidine with a modification at the C-5 position. C-5 modified pyrimidines can enhance the nuclease resistance of oligonucleotides, and are known in the art. For example, reference can be made to PCT Application WO 2011/130195 and references cited therein. In some preferred embodiments, the modification is a 2'-methoxy (2'-OMe) modification. In some embodiments, the modification, such as 2'-methoxy (2'-OMe) modification, is made to one or more nucleotides, for example 4 nucleotides, at the 5' and/or 3' end of the aptamer.

The modifications further include internucleotide modifications, such as those internucleotide modifications with uncharged bonds (such as methyl phosphonate, phosphotriester, phosphoamine ester, carbamate, etc.) and those internucleotide modifications with charged bonds (such as phosphorothioate, phosphorodithioate, etc.), internucleotide modifications with intercalating agents (such as acridine, psoralen, etc.), internucleotide modifications containing chelating agents (such as metals, radioactive metals, boron, oxidizing metals, etc.), internucleotide modifications containing alkylating agents, and internucleotide modifications with modified bonds (for example, alpha anomeric nucleic acid, etc.).

The modification may also include pegylation modification (PEG modification). Conjugation with PEG can extend the half-life of the aptamer, such as the *in vivo* half-life. In some embodiments, the molecular weight of the PEG is about 1 kDa to about 100 kDa, for example, about 10 kDa to about 80 kDa, about 20 kDa to about 60 kDa, about 30 kDa to about 50 kDa, about 40 kDa. In some embodiments, the PEG may be conjugated to the 5' end of the aptamer. In some embodiments, the PEG may be conjugated to the 3' end of the aptamer.

The modification may further include a fatty acid modification and/or a coumarin modification. For example, the aptamer may be conjugated with a fatty acid and/or a coumarin derivative (for example, conjugated to the 5' end of the aptamer molecule). The suitable fatty acid includes but not limited to dodecanedioic acid, palmitic acid (PA), tetradecanedioic acid, hexadecanedioic acid, stearic acid (SA), octadecanedioic acid, lauric acid, eicosapentaenoic acid (EPA), docosahexaenoic acid (DHA), and arachidonic acid (ARA). The suitable coumarin derivative includes but not limited to 4-hydroxycoumarin, 3-acetyl-6-carboxycoumarin, warfarin, (2-oxo-2H-chromene-3-yl) acetic acid, [8-acetyl-4-methyl-2-oxo-2H-chromene-7-yl) oxygen] acetic acid, coumarin-3-carboxylic acid, N-(4-methyl-7-coumarin) oxalamide, 7-(carboxymethyl)-4-methylcoumarin, 7-methoxycoumarin-3-carboxylic acid, and 6-methoxy-2-oxo-2H-chromene-3-carboxylic acid.

In some embodiments, the aptamer may comprise a combination of various modifications described above. For example, the aptamer may include 2'-methoxy (2'-OMe) modification and 3' inverted deoxythymidine (3' idT) modification.

In some embodiments, the *in vivo* half-life of the modified aptamer of the present invention is at least about 2 times, at least about 5 times, at least about 10 times, at least about 25 times, at least about 50 times, at least about 100 times, at least about 200 times or more times of the *in vivo* half-life of the corresponding unmodified aptamer.

### Treatment of diseases

In another aspect, the present invention provides a method for treating diseases by the aptamer against DKK1 of the present invention, the method comprises administering a therapeutically effective amount of the aptamer against DKK1 of the present invention to a subject in need thereof.

The diseases treated by the aptamer against DKK1 of the present invention are, for example, DKK1-related diseases, such as DKK1-mediated diseases. In an embodiment, the DKK1 related disease is characterized by a disease with abnormal expression of DKK1, such as overexpression of DKK1.

As used herein, "a DKK1-related disease" includes DKK1-related cancers. In an embodiment, "DKK1-related cancer" is characterized by a cancer with abnormal expression of DKK1, such as overexpression of DKK1. In an embodiment, DKK1-related cancer is selected from, for example, myeloma (e.g., multiple myeloma with osteolytic lesions, hilar cholangiocarcinoma multiple myeloma), breast cancer, colon cancer, melanoma, hepatocellular cancer, epithelial cancer, esophageal cancer, gastric cancer, gastroesophageal cancer, hilar cholangiocarcinoma, brain cancer, lung cancer, prostate cancer, or pancreatic cancer, as well as any metastases thereof.

As used herein, "a DKK1-related disease" includes a bone-related disease, such as the bone-related disease related to abnormal osteoblast or osteoclast activity. The disease characterized by the abnormal osteoblast or osteoclast activity includes but not limited to: primary and secondary osteoporosis, osteopenia, osteomalacia, osteogenesis imperfecta (OI), avascular necrosis (osteonecrosis), fractures and implant healing (dental implants and hip implants), bone loss due to other diseases (e.g., associated with HIV infection, cancers, or arthritis). Other "DKK1-related diseases" include but are not limited to rheumatoid arthritis, osteoarthritis, arthritis, and osteolytic lesions.

The subject can be any animal (domesticated, domestic animal or wild), including but not limited to cats, dogs, horses, pigs, and cattles, and human subjects are preferred. As used herein, the terms patient, individual, and subject can be used interchangeably. In some embodiment, the subject can be a male or a female.

As used herein, "treating" an individual suffering from a disease means that the individual's symptoms are partially or completely alleviated, or remain unchanged after treatment. Thus, treatment includes prevention, treatment and/or cure. Prevention refers to prevention of a potential disease and/or prevention of worsening of symptoms or disease progression.

As used herein, "therapeutically effective amount" or "therapeutically effective dose" refers to an amount of a substance, compound, material, or composition comprising a compound that is at least sufficient to produce a therapeutic effect after administration to a subject. Thus, it is the amount necessary to prevent, cure, ameliorate, arrest or partially arrest the symptoms of the disease or condition. As used herein, "therapeutic effect" means an effect resulting from treatment of an individual that alters, generally ameliorates or alleviates the symptoms of the disease or disease condition, or cures the disease or disease condition.

The dosage regimen of the aptamer against DKK1 is selected according to a variety of factors, including, for example, the type, species, age, weight, gender, and medical condition of the patient; the severity of the condition to be treated; the route of administration; the kidney function and liver function of the patient; and the specific aptamer against DKK1 or salt thereof as used. Ordinary skilled doctors can easily determine and specify the effective amount of the composition required to prevent, combat, or inhibit the progression of the condition.

Typically, the dosage regimen of the aptamer against DKK1 is about 1 µg/kg body weight to about 100 mg/kg body weight per day.

An exemplary treatment regime entails administration once daily, once every two days, once per week, twice per week, once every two weeks, once every three weeks, once every four weeks, once a month, once every 3 months or once every three to 6 months, or with a short administration interval at the beginning (such as once per week to once every three weeks), and then an extended interval later (such as once a month to once every three to 6 months). The frequency and interval of administration can be determined by those skilled in the art according to the pharmacokinetic parameters of the aptamer.

### Pharmaceutical composition

In another aspect, the present invention also provides a pharmaceutical composition comprising at least one aptamer against DKK1 of the present invention, and a pharmaceutically acceptable carrier or excipient. Said pharmaceutical composition is used for treating, for example, DKK1-related diseases.

The aptamers described herein can be utilized in any pharmaceutically acceptable dosage form, including but not limited to injectable dosage forms, liquid dispersions, gels, aerosols, ointments, creams, lyophilized formulations, dry powders, tablets, capsules, controlled release formulations, fast melt formulations, delayed release formulations, extended release formulations, pulsatile release formulations, mixed immediate release and controlled release formulations, etc. Specifically, the aptamers described herein can be formulated: (a) for administration selected from any of oral, pulmonary, intravenous, intra-arterial, intrathecal, intraarticular, rectal, ophthalmic, colonic, parenteral, intracisternal, intravaginal, intraperitoneal, local, buccal, nasal, and topical administration; (b) into a dosage form selected from any of liquid dispersions, gels, aerosols, ointments, creams, tablets, sachets and capsules; (c) into a dosage form selected from any of lyophilized formulations, dry powders, fast melt formulations, controlled release formulations, delayed release formulations, extended release formulations, pulsatile release formulations, and mixed immediate release and controlled release formulations; or (d) any combination thereof.

Solutions or suspensions used for parenteral, intradermal, or subcutaneous application can comprise one or more of the following components: (1) a sterile diluent such as water for injection, saline solution, fixed oils, polyethylene glycols, glycerin, propylene glycol or other synthetic solvents; (2) antibacterial agents such as benzyl alcohol or methyl parabens; (3) antioxidants such as ascorbic acid or sodium bisulfite; (4) chelating agents such as ethylenediaminetetraacetic acid; (5) buffers such as acetates, citrates or phosphates; and (6) agents for the adjustment of tonicity such as sodium chloride or dextrose. The pH can be adjusted with acids or bases, such as hydrochloric acid or sodium hydroxide. A parenteral preparation can be enclosed in ampoules, disposable syringes or multiple dose vials made of glass or plastic.

Pharmaceutical compositions suitable for injectable use may include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition should be sterile and should be fluid to the extent that easy syringability exists. The pharmaceutical composition should be stable under the conditions of manufacture and storage and should be preserved against the contaminating action of microorganisms such as bacteria and fungi. The term "stable", as used herein, means remaining in a state or condition that is suitable for administration to a patient.

The carrier can be a solvent or dispersion medium, including, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol or sorbitol, and inorganic salts such as sodium chloride in the composition. Prolonged absorption of the injectable compositions can be brought about by including in the composition an agent which delays absorption, for example, aluminum monostearate and gelatin.

Sterile injectable solutions can be prepared by incorporating the active reagent (e.g., an aptamer against DKK1) in the required amount in an appropriate solvent with one or a combination of ingredients enumerated above, as required, followed by filtered sterilization. Generally, dispersions are prepared by incorporating at least one aptamer against DKK1into a sterile vehicle that contains a basic dispersion medium and any other required ingredient. In the case of sterile powders for the preparation of sterile injectable solutions, exemplary preparation methods include vacuum drying and freeze-drying, both of which will yield a powder of the aptamer against DKK1 and any additional desired ingredient from a previously sterile-filtered solution thereof.

Oral compositions generally include an inert diluent or an edible carrier. They can be enclosed, for example, in gelatin capsules or compressed into tablets. For the purpose of oral therapeutic administration, the aptamer against DKK1 can be incorporated with excipients and used in the form of tablets, troches, or capsules. Pharmaceutically compatible binding agents, and/or adjuvant materials can be included as part of the composition.

For administration by inhalation, the compounds are delivered in the form of an aerosol spray from a pressured container or dispenser that contains a suitable propellant, e.g., a gas such as carbon dioxide, a nebulized liquid, or a dry powder from a suitable device. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active reagents are formulated into ointments, salves, gels, or creams as generally known in the art. The reagents can also be prepared in the form of suppositories (e.g., with conventional suppository bases such as cocoa butter and other glycerides) or retention enemas for rectal delivery.

In one embodiment, the aptamer against DKK1 is formulated for topical administration. As used herein "topical administration" refers to the delivery of an aptamer against DKK1 to an animal by contacting, directly or otherwise, a formulation comprising the aptamer against DKK1 to all or a portion of the skin (epidermis) of an animal. The term encompasses several routes of administration including, but not limited to, topical and transdermal. A common requirement for these modes of administration is efficient delivery to the target tissue or stratum. In one aspect, topical administration is used as a means to penetrate the epidermis and dermis and ultimately achieve systemic delivery of the aptamer against DKK1. In another aspect, topical administration is used as a means to selectively deliver the aptamer against DKK1 to the epidermis or dermis of an animal, or to specific strata thereof.

For topical administration, the aptamer against DKK1 may be formulated into pharmaceutically acceptable ointments, creams, lotions, eye ointments, eye drops, ear drops, impregnated dressings, and aerosols, medicated powders, medicated adhesives, foams, and may contain appropriate conventional additives or excipients, including, for example, preservatives or solvents to assist drug penetration, and emollients in ointments, gels, and creams. Such topical formulations may also contain compatible conventional carriers, for example ethanol or oleyl alcohol for lotions. Such carriers may constitute from about 1% to about 98% by weight of the formulation; more usually, such carriers will constitute up to about 80% by weight of the formulation. Specific formulations for the topical delivery of aptamers are described in the art.

In one embodiment, an aptamer against DKK1 is prepared with a carrier that will protect against rapid elimination from the body. For example, a controlled release formulation can be used, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions, including liposomes with monoclonal antibodies against viral antigens that target the infected cells, can also be used as pharmaceutically acceptable carriers. These can be prepared according to methods known to those skilled in the art.

Additionally, suspensions of the aptamer against DKK1 may be prepared as appropriate oily injection suspensions. Suitable lipophilic solvents or vehicles include fatty oils, such as sesame oil, or synthetic fatty acid esters, such as ethyl oleate, triglycerides, or liposomes. Nonlipid polycationic amino polymers may also be used for delivery. Optionally, the suspension may also include suitable stabilizers or agents to increase the solubility of the compounds and allow for the preparation of highly concentrated solutions.

In some cases, it may be especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of a aptamer against DKK1 calculated to produce the desired therapeutic effect in association with the required pharmaceutical carrier. The specification for the dosage unit forms of the aptamer against DKK1 described herein are dictated by and directly dependent on the unique characteristics of the particular aptamer against DKK1 and the particular therapeutic effect to be achieved, and the limitations inherent in the art of compounding such an active agent for the treatment of individuals.

Pharmaceutical compositions comprising at least one aptamer against DKK1 can include one or more pharmaceutical excipients. Examples of such excipients include, but are not limited to, binding agents, filling agents, lubricating agents, suspending agents, sweeteners, flavoring agents, preservatives, buffers, wetting agents, disintegrants, effervescent agents, and other excipients. Such excipients are known in the art. Exemplary excipients include: (1) binding agents which include various celluloses and cross-linked polyvinylpyrrolidone, microcrystalhne cellulose, such as Avicel PH101 and Avicel PH102, silicified microcrystalhne cellulose (ProSolv SMCC^{™}), gum tragacanth and gelatin; (2) filling agents such as various starches, lactose, lactose monohydrate, and lactose anhydrous; (3) disintegrating agents such as alginic acid, Primogel, corn starch, lightly crosslinked polyvinyl pyrrolidone, potato starch, maize starch, and modified starches, croscarmellose sodium, cross-povidone, sodium starch glycolate, and mixtures thereof; (4) lubricants, including agents that act on the flowability of a powder to be compressed, include magnesium stearate, colloidal silicon dioxide, such as Aerosil 200, talc, stearic acid, calcium stearate, and silica gel; (5) glidants such as colloidal silicon dioxide; (6) preservatives, such as potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride; (7) diluents such as pharmaceutically acceptable inert fillers, such as microcrystalhne cellulose, lactose, dibasic calcium phosphate, saccharides, and/or mixtures of any of the foregoing; examples of diluents include microcrystalline cellulose, such as Avicel PH101 and Avicel PH102; lactose such as lactose monohydrate, lactose anhydrous, and Pharmatose DCL21; dibasic calcium phosphate such as Emcompress; mannitol; starch; sorbitol; sucrose; and glucose; (8) sweetening agents, including any natural or artificial sweetener, such as sucrose, saccharin sucrose, xylitol, sodium saccharin, cyclamate, aspartame, and acesulfame; (9) flavoring agents, such as peppermint, methyl salicylate, orange flavoring, Magnasweet (trademark of MAFCO), bubble gum flavor, fruit flavors, and the like; and (10) effervescent agents, including effervescent couples such as an organic acid and a carbonate or bicarbonate.

### Examples

The present invention is further described by the following examples, but the scope of the present invention is not limited to the described examples.

### Material and method

**Protein-SELEX:** The aptamers against DKK1 were screened by a SELEX method based on Ni-NTA beads. In short, His6-tagged DKK-1 was firstly incubated with the Ni-NTA beads, to form protein bound Ni-NTA magnetic beads. Then, such complex was incubated with an ssDNA random library at room temperature to form an ssDNA-protein-bound Ni-NTA magnetic bead complex. After being washed, the ssDNA-protein-bound Ni-NTA magnetic bead complex is used as a template for performing PCR with a forward primer and a biotinylated reverse primer. According to the manufacturer's protocol, ssDNA was regenerated by magnetic beads coated with streptavidin. The ssDNA pool was used for the next round of SELEX. From the 4th round, the evolved ssDNA pool was incubated with Ni-NTA beads or non-target proteins for reverse selection. Then, unbound ssDNA was collected from the reverse selection and negative selection and applied to DKK1-bound Ni-NTA magnetic beads. ssDNA products from rounds 10, 16, 18, and 20 were used to determine the degree of enrichment in the selection process compared to the initial random library. The ssDNA library with the highest binding affinity was cloned and sequenced.

**Enzyme linked oligonucleotide assay (ELONA):** 160 ng DKK1 per well and negative target were coated in a 96-well microtiter plate by incubating at 4°C overnight through hydrophobic interaction. Non-binding sites in wells were blocked with bull serum albumin (BSA) at room temperature for 1 hour, and then washed with DNA-binding buffer 4 times for 5 minutes each. The biotinylated aptamer candidates/RS with an appropriate concentration were added to each well and incubated at room temperature for 45 minutes with continuous gentle shaking. After binding, the plate was washed with washing buffer 4 times for 5 minutes each to remove non-specific and weakly binding sequences. 100 µl streptavidin-horseradish peroxidase (HRP) (1:10000 dilute into PBST+0.1% BSA) was added to each well, and incubated for 30 minutes, and washed for 4 times with the binding buffer. 50 µl 3,3',5,5'-tetramethylbenzidine (TMB) as a HRP substrate was added to each well and incubated for 20 minutes. 50 µl 2M H₂SO₄ was added to terminate the reaction. The absorbance at 450 nm was measured with a microplate reader (Molecular Device i3x). Data was analyzed with Origin software. A binding curve was generated and K_{D} was calculated with a non-linear curve fitting model logistic.

**TCF-wnt induced luciferase activity assay:** HEK293T cells were co-transfected with corresponding plasmids, including TOPFlash, FOPFlash, Wnt3a, and DKK1 constructs. An equal amount of the carrier was used for each time of transfection. The culture medium was replaced 24 hours after the transfection. The cells were treated with conditional culture medium containing 1 µM corresponding aptamer candidates. In order to determine inhibition effect, the cells are treated with aptamer candidates having gradient concentrations. 24 hours after treatment, the cells were lysed with 100 µl of lysis buffer, and 20 µl was taken for analysis. It was calculated according to the protocol (dual luciferase reporter gene assay, Promega).

**Binding ability assay:** 48 h after the transfection, the culture medium was collected in a 15 ml centrifuge tube and centrifuged at 3000 rpm for 10 min. 2 ml of a supernatant was incubated with 30 µl NTA magnetic beads at room temperature for 1 hour (Saiyed, Telang et al., 2003). Unbound proteins may be removed by washing the beads twice with PBS. Afterwards, the bead-DKK1 complex was incubated with 250 nM FAM-tagged aptdkk5s in a total volume of 20 µl at room temperature for 45 min. After the incubation, the bead complex was washed twice with PBS to remove unbound DNA. Then, the fluorescence signal was detected under an inverted fluorescence microscope.

**DNA detection:** the band density of all aptamer samples was measured by a molecular imager (Bio-Rad) (Klussmann, Nolte et al. 1996, Siller-Matula, Merhi et al. 2012).

### Example 1: Recombinant His6-DKK1 immobilized on NTA magnetic beads

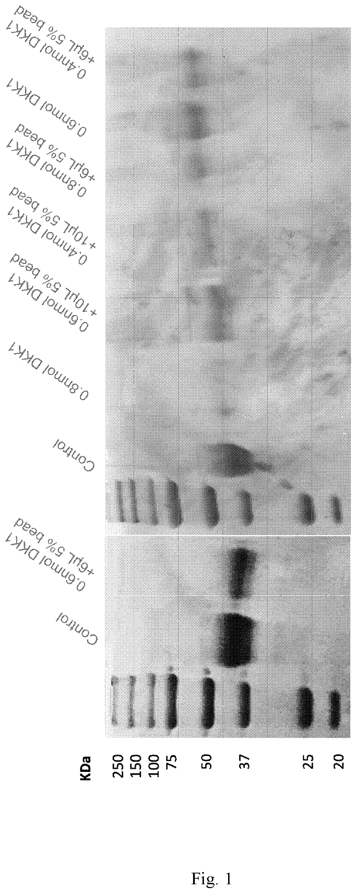

Ni-NTA magnetic agarose beads were provided in a 5% (v/v) suspension, its binding ability to 6x His-tagged proteins was 300 µg protein (~24 kDa) per milliliter of the suspension. In order to immobilize 0.2 nmol of DKK1 on the magnetic beads in the subsequent first round of SELEX, the amount and ratio of the recombinant His6-DKK1 and NTA magnetic beads were optimized. 0.8 nmol protein + 10 µl magnetic beads, 0.6 nmol protein + 10 µl magnetic beads, 0.4 nmol protein + 10 µl magnetic beads, 0.8 nmol protein + 6 µl magnetic beads, 0.6 nmol protein + 6 µl magnetic beads, and 0.4 nmol protein + 6 µl magnetic beads were incubated at 4°C for 60 min. After the incubation, the protein-bead complex was analyzed by SDS-PAGE. According to results, the optimal condition was 0.6 nmol His6-DKK1 and 6 µL NTA agarose magnetic beads (Fig. 1). Therefore, such condition was used for the following study.

### Example 2: SELEX of aptamer targeting full-length DKK1

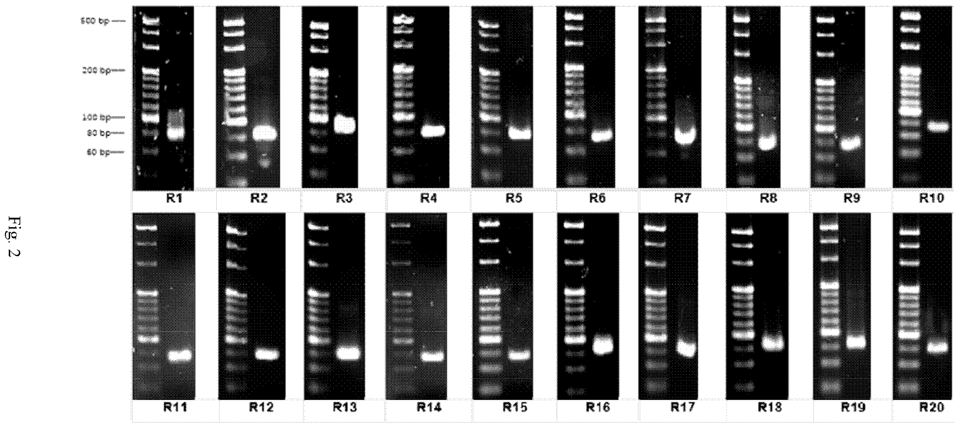

Recombinant human DKK-1 with a His6 tag was immobilized on Ni-NTA magnetic beads and used as a forward selection target. Blank Ni-NTA beads and other proteins with a His6 tag were used as a negative selection target. The ssDNA pool collected after each round of forward selection was amplified by PCR and detected by agarose gel electrophoresis before ssDNA regeneration (Fig. 2). The PCR cycle was monitored to avoid non-specific byproducts in PCR. 20 rounds of SELEX were performed.

### Example 3: Analysis of sequencing data

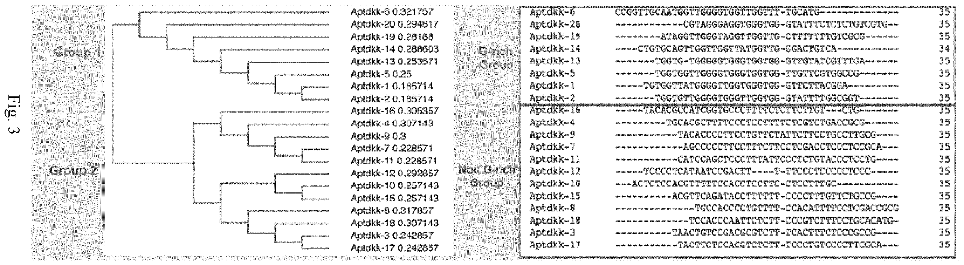

The 10th round and final round of the ssDNA pools were sequenced by Next Generation Sequencing. There was no enriched sequence in the 10th round, while the enriched sequence appeared in the final round (Table 1). Therefore, 20 potential sequences with the highest occurrence rate were identified in the finally enriched ssDNA pool and named Aptdkk-1-20 (Table 2). According to multi-sequence alignment results using Cluster Omega, these sequences might be divided into two groups (Fig. 3). The first group was apparently rich in guanine (shown in a red box), while the second group was a non-G rich sequence (shown in a blue box).

**Table 1: Enrichment results from final round**

| Enrichment | Sequence quantity |
|---|---|
| 15 | 1 |
| 6 | 2 |
| 5 | 3 |
| 4 | 6 |
| 3 | 21 |
| 2 | 395 |

**Table 2: Sequences with highest enrichment from final round (excluding the conserved primer region)**

| SE Q ID NO | Correspondi ng aptamer | Enrichme nt | Sequence |
|---|---|---|---|
| 45 | Aptdkk-1 | 15 | TGTGGTTATGGGGTTGGTGGGTGGGTTCTTAC GGA |
| 46 | Aptdkk-2 | 6 | TGGTGTTGGGGTGGGTTGGTGGGTATTTTGGC GGT |
| 47 | Aptdkk-3 | 6 | TAACTGTCCGACGCGTCTTTCACTTTCTCCCG CCG |
| 48 | Aptdkk-4 | 5 | TGCACGCTTTTCCCTCCTTTTCTCGTCTGACC GCG |
| 49 | Aptdkk-5 | 5 | TGGTGGTTGGGGTGGGTGGTGGTTGTTCGTG GCCG |
| 50 | Aptdkk-6 | 5 | CCGGTTGCAATGGTTGGGGTGGTTGGTTTTGC ATG |
| 51 | Aptdkk-7 | 4 | AGCCCCCTTCCTTTCTTCCTCGACCTCCCTCC GCA |
| 52 | Aptdkk-8 | 4 | TGCCACCCCTGTTTTCCACATTTTCCTCGACC GCG |
| 53 | Aptdkk-9 | 4 | TACACCCCTTCCTGTTCTATTCTTCCTGCCTTG CG |
| 54 | Aptdkk-10 | 4 | ACTCTCCACGTTTTTCCACCTCCTTCCTCCTTT GC |
| 55 | Aptdkk-11 | 4 | CATCCAGCTCCCTTTATTCCCTCTGTACCCTCC TG |
| 56 | Aptdkk-12 | 4 | TCCCCTCATAATCCGACTTTTTCCCTCCCCCTC CC |
| 57 | Aptdkk-13 | 3 | TGGTGTGGGGGTGGGTGGTGGGTTGTATCGTT TGA |
| 58 | Aptdkk-14 | 3 | CTGTGCAGTTGGTTGGTTATGGTTGGGACTGT CA |
| 59 | Aptdkk-15 | 3 | ACGTTCAGATACCTTTTTTCCCCTTTGTTCTGC CG |
| 60 | Aptdkk-16 | 3 | TACACGCCATCGGTGCCCTTTTCTCTTCTTGTC TG |
| 61 | Aptdkk-17 | 3 | TACTTCTCCACGTCTCTTTCCCTGTCCCCTTCG CA |
| 62 | Aptdkk-18 | 3 | TCCACCCAATTCTCTTCCCGTCTTTCCTGCACA TG |
| 63 | Aptdkk-19 | 3 | ATAGGTTGGGTAGGTTGGTTGCTTTTTTTGTC GCG |
| 64 | Aptdkk-20 | 3 | CGTAGGGAGGTGGGTGGGTATTTCTCTCTGTC GTG |

### Example 4: Prediction of G-group mode

G-rich sequences typically formed a special G-quadruplex structure, which had a stable helical shape, and contained a guanine tetrad that might be formed by one, two, or four chains. Then, the G-rich sequences were analyzed by QGRS of potential G groups and G scores of their tendency to form a single molecular quadruplex. Usually, the size and distribution of gaps in predicted QGRS were considered when the G scores were calculated. The sequence with higher score might become a better candidate for the G-quadruplex. According to analysis results, all these sequences had two G tetrads, which might form two G chains (Table 3). The G scores were all around 19-20. In addition, the distribution of the gaps was shown in a similar pattern. It was indicated that their ability to form the stable G-quadruplex was very similar.

**Table 3: QGRS analysis of G-rich groups**

| **The sequences of this group showed similarity in length and G score of QGRS.** | | | |
|---|---|---|---|
| Name | Length | QGRS | G-score |
| Aptdkk-6 | 15 | GGTTGGGGTGGTTGG | 20 |
| Aptdkk-20 | 14 | GGGAGGTGGGTGGG | 21 |
| Aptdkk-19 | 15 | GGTTGGGTAGGTTGG | 20 |
| Aptdkk-14 | 16 | GGTTGGGTAGGTTGG | 19 |
| Aptdkk-13 | 14 | GGGTGGGTGGTGGG | 21 |
| Aptdkk-5 | 18 | GGTTGGGGTGGGTGGTGG | 20 |
| Aptdkk-1 | 14 | GGTTGGTGGGTGGG | 21 |
| Aptdkk-2 | 14 | GGTGGGTTGGTGGG | 21 |

### Example 5: Specificity of representative aptamer candidates

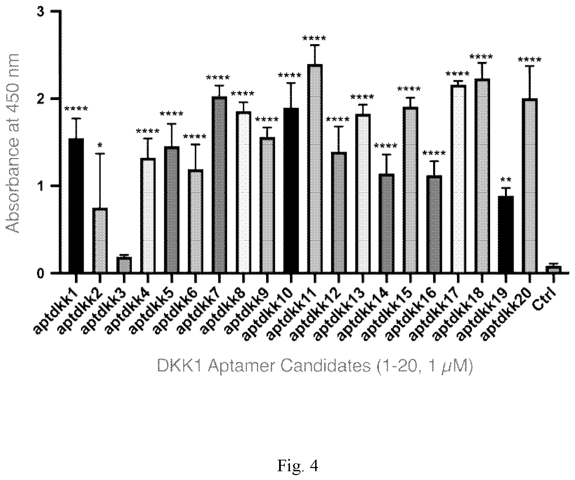

The specificity of 1 µM of each adapter/RS against DKK-1 was determined for 20 potential sequences with the highest occurrence rate (Table 4) by ELONA and the absorbance at 450 nm was detected. Compared with a blank control, most of aptamer candidates except for Candidate 3 showed the significantly higher absorbance (Fig. 4).

**Table 4: 20 potential candidates (including the conserved primer region)**

| SEQ ID NO: | Name | Sequence | Leng th (nt) |
|---|---|---|---|
| 1 | Aptdkk-1 | CGTACGGTCGACGCTAGCTGTGGTTATGGGGTT GGTGGGTGGGTTCTTACGGAGGATCCGAGCTCC ACGTG | 71 |
| 2 | Aptdkk-2 | CGTACGGTCGACGCTAGCTGGTGTTGGGGTGGG TTGGTGGGTATTTTGGCGGTGGATCCGAGCTCCA CGTG | 71 |
| 3 | Aptdkk-3 | CGTACGGTCGACGCTAGCTAACTGTCCGACGCG TCTTTCACTTTCTCCCGCCGGGATCCGAGCTCCA CGTG | 71 |
| 4 | Aptdkk-4 | CGTACGGTCGACGCTAGCTGCACGCTTTTCCCTC CTTTTCTCGTCTGACCGCGGGATCCGAGCTCCAC GTG | 71 |
| 5 | Aptdkk-5 | CGTACGGTCGACGCTAGCTGGTGGTTGGGGTGG GTGGTGGTTGTTCGTGGCCGGGATCCGAGCTCC ACGTG | 71 |
| 6 | Aptdkk-6 | CGTACGGTCGACGCTAGCCCGGTTGCAATGGTT GGGGTGGTTGGTTTTGCATGGGATCCGAGCTCC ACGTG | 71 |
| 7 | Aptdkk-7 | CGTACGGTCGACGCTAGCAGCCCCCTTCCTTTCT TCCTCGACCTCCCTCCGCAGGATCCGAGCTCCA | 71 |
| | | CGTG | |
| 8 | Aptdkk-8 | CGTACGGTCGACGCTAGCTGCCACCCCTGTTTTC CACATTTTCCTCGACCGCGGGATCCGAGCTCCA CGTG | 71 |
| 9 | Aptdkk-9 | CGTACGGTCGACGCTAGCTACACCCCTTCCTGTT CTATTCTTCCTGCCTTGCGGGATCCGAGCTCCAC GTG | 71 |
| 10 | Aptdkk-10 | CGTACGGTCGACGCTAGCACTCTCCACGTTTTTC CACCTCCTTCCTCCTTTGCGGATCCGAGCTCCAC GTG | 71 |
| 11 | Aptdkk-11 | CGTACGGTCGACGCTAGCCATCCAGCTCCCTTTA TTCCCTCTGTACCCTCCTGGGATCCGAGCTCCAC GTG | 71 |
| 12 | Aptdkk-12 | CGTACGGTCGACGCTAGCTCCCCTCATAATCCGA CTTTTTCCCTCCCCCTCCCGGATCCGAGCTCCAC GTG | 71 |
| 13 | Aptdkk-13 | CGTACGGTCGACGCTAGCTGGTGTGGGGGTGGG TGGTGGGTTGTATCGTTTGAGGATCCGAGCTCCA CGTG | 71 |
| 14 | Aptdkk-14 | CGTACGGTCGACGCTAGCCTGTGCAGTTGGTTG GTTATGGTTGGGACTGTCAGGATCCGAGCTCCA CGTG | 70 |
| 15 | Aptdkk-15 | CGTACGGTCGACGCTAGCACGTTCAGATACCTTT TTTCCCCTTTGTTCTGCCGGGATCCGAGCTCCAC GTG | 71 |
| 16 | Aptdkk-16 | CGTACGGTCGACGCTAGCTACACGCCATCGGTG CCCTTTTCTCTTCTTGTCTGGGATCCGAGCTCCA CGTG | 71 |
| 17 | Aptdkk-17 | CGTACGGTCGACGCTAGCTACTTCTCCACGTCTC TTTCCCTGTCCCCTTCGCAGGATCCGAGCTCCAC GTG | 71 |
| 18 | Aptdkk-18 | CGTACGGTCGACGCTAGCTCCACCCAATTCTCTT CCCGTCTTTCCTGCACATGGGATCCGAGCTCCAC GTG | 71 |
| 19 | Aptdkk-19 | CGTACGGTCGACGCTAGCATAGGTTGGGTAGGT TGGTTGCTTTTTTTGTCGCGGGATCCGAGCTCCA CGTG | 71 |
| 20 | Aptdkk-20 | CGTACGGTCGACGCTAGCCGTAGGGAGGTGGGT GGGTATTTCTCTCTGTCGTGGGATCCGAGCTCCA CGTG | 71 |

### Example 6: Affinity of representative aptamer candidates

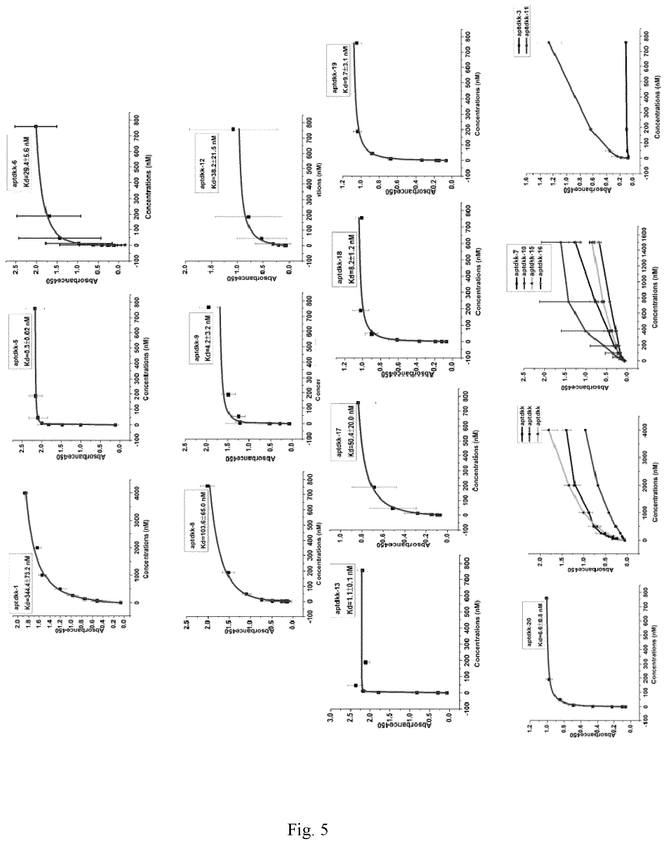

Aptamer candidates with significant specificity and Candidate 3 as a negative control were used to determine the binding affinity against DKK1. Candidates 1, 2, 4, and 14 had concentrations of (0, 62.5 nM, 125 nM, 250 nM, 500 nM, 1000 nM, 2000 nM, and 4000 nM), Candidates 7, 10, 15, and 16 had concentrations of (0, 23.44 nM, 46.86 nM, 93.75 nM, 187.5 nM, 375 nM, 750 nM, and 1500 nM), and other candidates had concentrations of (0, 0.18 nM, 0.74 nM, 2.96 nM, 11.84 nM, 47.35 nM, 189.39 nM, and 757.58 nM). The binding curve of each candidate was shown in Fig. 5 and Table 5. 11 aptamer candidates showed the high affinity (low K_{d} value) against DKK1. Six candidates (aptdkk-5, 13, 20, 19, 6, and 1) were rich in G. The other five sequences (aptdkk-9, 18, 12, 17, and 8) were not rich in G.

**Table 5: Summary of specificity and affinity of representative aptamer candidates**

| | Aptamer | Specificity | K_{d} value(nM) |
|---|---|---|---|
| G rich group | Aptdkk-5 | High | 0.3 ± 0.02 |
| | Aptdkk-13 | High | 1.1 ± 0.1 |
| | Aptdkk-20 | High | 6.6 ± 0.8 |
| | Aptdkk-19 | High | 9.7 ± 3.1 |
| | Aptdkk-6 | High | 29.4 ± 5.6 |
| | Aptdkk-1 | High | 344.4 ± 73.2 |
| | Aptdkk-2 | High | - |
| | Aptdkk-14 | High | - |
| Non-G rich group | Aptdkk-9 | High | 4.2 ± 3.2 |
| | Aptdkk-18 | High | 8.2 ± 1.2 |
| | Aptdkk-12 | High | 38.2 ± 21.5 |
| | Aptdkk-17 | High | 50.4 ± 20 |
| | Aptdkk-8 | High | 103.6 ± 65 |
| | Aptdkk-3 | Low | - |
| | Aptdkk-4 | High | - |
| | Aptdkk-7 | High | - |
| | Aptdkk-10 | High | - |
| | Aptdkk-11 | High | - |
| | Aptdkk-15 | High | - |
| | Aptdkk-16 | High | - |

### Example 7: Inhibition effect of representative aptamer candidates

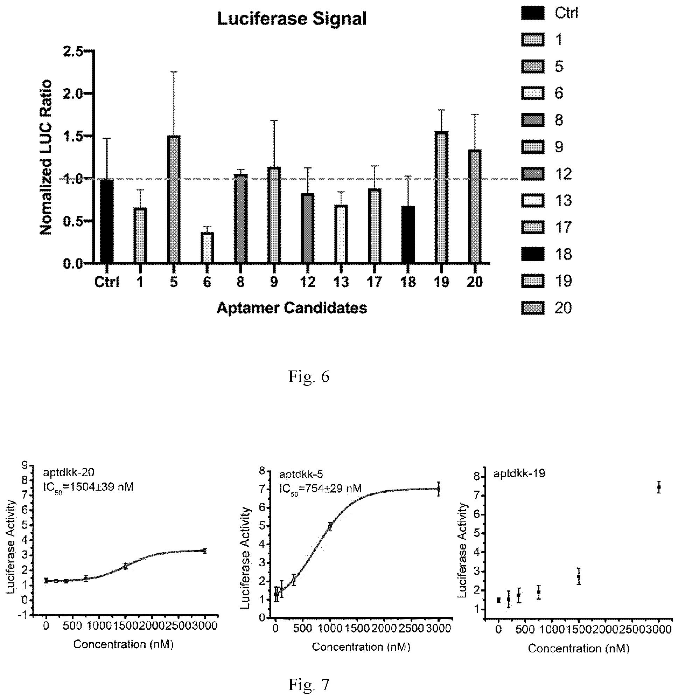

Compared with the untreated group, cells treated with aptdkk-5, 19, and 20 showed higher luciferase signals, indicating that they might release inhibition of Wnt signaling by DKK1 (Fig. 6). However, cells treated with other aptamer candidates showed luciferase signals similar to the untreated group. Therefore, aptdkk-5, 19, and 20 might inhibit the antagonistic effect of DKK1 on Wnt signaling.

### Example 8: Inhibition efficacy of representative aptamer candidates

According to calculation, the inhibition efficacy of aptdkk-5 was IC₅₀=754 ± 29 nM, and the inhibition efficacy of aptdkk-20 was IC₅₀=1504 ± 39 nM. Data of Candidate 19 did not fit to the fitting curve of the tested concentrations, indicating that a higher IC₅₀ (Fig. 7). This result indicated that aptdkk-5 and aptdkk-20 both showed the inhibition effects on the inhibition of Wnt signaling by DKK1, while the effect of aptdkk-19 was relatively weak.

### Example 9: Specificity of truncated aptdkk-5

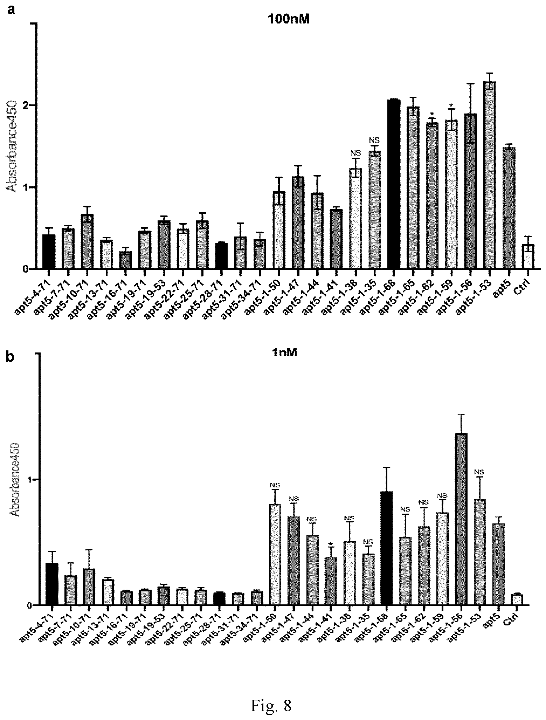

The specificity of 100 nM/1 nM of each aptamer/RS against DKK-1 was determined for 24 truncated sequences of aptdkk5 and original aptdkk5 (Table 6) by ELONA and the absorbance at 450 nm was detected. When the concentration was 100 nM, there was no significant difference or slight difference in the absorbance between apt5-1-38, apt5-1-35, apt5-1-62, or apt5-1-59 and the original aptdkk5 (Fig. 8a). When the concentration was 1 nM, there was no significant difference or slight difference in the absorbance between apt5-1-50, apt5-1-47, apt5-1-44, apt5-1-41, apt5-1-38, apt5-1-35, apt5-1-65, apt5-1-62, apt5-1-59, or apt5-1-53 and the original aptdkk5 (Fig. 8b). After the specificity and length of these aptamers were considered, apt5-1-44, apt5-1-41, apt5-1-38, and apt5-1-35 were selected to determine the binding affinity against DKK1.

**Table 6: Truncated sequences of Aptdkk-5**

| **SEQ ID NO:** | **Name** | **Sequence (5' to 3')** | **Lengt h(nt)** | **Nucle otides** |
|---|---|---|---|---|
| 21 | Apt5-4-71 | ACGGTCGACGCTAGCTGGTGGTTGGG GTGGGTGGTGGTTGTTCGTGGCCGCG TACGGTCGACGCTACG | 68 | 4-71 |
| 22 | Apt5-7-71 | GTCGACGCTAGCTGGTGGTTGGGGTG GGTGGTGGTTGTTCGTGGCCGCGTAC GGTCGACGCTACG | 65 | 7-71 |
| 23 | Apt5-10-71 | GACGCTAGCTGGTGGTTGGGGTGGGT GGTGGTTGTTCGTGGCCGCGTACGGT CGACGCTACG | 62 | 10-71 |
| 24 | Apt5-13-71 | GCTAGCTGGTGGTTGGGGTGGGTGGT GGTTGTTCGTGGCCGCGTACGGTCGA | 59 | 13-71 |
| | | CGCTACG | | |
| 25 | Apt5-16-71 | AGCTGGTGGTTGGGGTGGGTGGTGGT TGTTCGTGGCCGCGTACGGTCGACGC TACG | 56 | 16-71 |
| 26 | Apt5-19-71 | TGGTGGTTGGGGTGGGTGGTGGTTGT TCGTGGCCGCGTACGGTCGACGCTAC G | 53 | 19-71 |
| 27 | Apt5-19-53 | TGGTGGTTGGGGTGGGTGGTGGTTGT TCGTGGCCG | 35 | 19-53 |
| 28 | Apt5-22-71 | TGGTTGGGGTGGGTGGTGGTTGTTCG TGGCCGCGTACGGTCGACGCTACG | 50 | 22-71 |
| 29 | Apt5-25-71 | TTGGGGTGGGTGGTGGTTGTTCGTGG CCGCGTACGGTCGACGCTACG | 47 | 25-71 |
| 30 | Apt5-28-71 | GGGTGGGTGGTGGTTGTTCGTGGCCG CGTACGGTCGACGCTACG | 44 | 28-71 |
| 31 | Apt5-31-71 | TGGGTGGTGGTTGTTCGTGGCCGCGT ACGGTCGACGCTACG | 41 | 31-71 |
| 32 | Apt5-34-71 | GTGGTGGTTGTTCGTGGCCGCGTACG GTCGACGCTACG | 38 | 34-71 |
| 33 | Apt5-1-50 | CGTACGGTCGACGCTAGCTGGTGGTT GGGGTGGGTGGTGGTTGTTCGTGG | 50 | 1-50 |
| 34 | Apt5-1-47 | CGTACGGTCGACGCTAGCTGGTGGTT GGGGTGGGTGGTGGTTGTTCG | 47 | 1-47 |
| 35 | Apt5-1-44 | CGTACGGTCGACGCTAGCTGGTGGTT GGGGTGGGTGGTGGTTGT | 44 | 1-44 |
| 36 | Apt5-1-41 | CGTACGGTCGACGCTAGCTGGTGGTT GGGGTGGGTGGTGGT | 41 | 1-41 |
| 37 | Apt5-1-38 | CGTACGGTCGACGCTAGCTGGTGGTT | 38 | 1-38 |
| | | GGGGTGGGTGGT | | |
| 38 | Apt5-1-35 | CGTACGGTCGACGCTAGCTGGTGGTT GGGGTGGGT | 35 | 1-35 |
| 39 | Apt5-1-68 | CGTACGGTCGACGCTAGCTGGTGGTT GGGGTGGGTGGTGGTTGTTCGTGGCC GCGTACGGTCGACGCT | 68 | 1-68 |
| 40 | Apt5-1-65 | CGTACGGTCGACGCTAGCTGGTGGTT GGGGTGGGTGGTGGTTGTTCGTGGCC GCGTACGGTCGAC | 65 | 1-65 |
| 41 | Apt5-1-62 | CGTACGGTCGACGCTAGCTGGTGGTT GGGGTGGGTGGTGGTTGTTCGTGGCC GCGTACGGTC | 62 | 1-62 |
| 42 | Apt5-1-59 | CGTACGGTCGACGCTAGCTGGTGGTT GGGGTGGGTGGTGGTTGTTCGTGGCC GCGTACG | 59 | 1-59 |
| 43 | Apt5-1-56 | CGTACGGTCGACGCTAGCTGGTGGTT GGGGTGGGTGGTGGTTGTTCGTGGCC GCGT | 56 | 1-56 |
| 44 | Apt5-1-53 | CGTACGGTCGACGCTAGCTGGTGGTT GGGGTGGGTGGTGGTTGTTCGTGGCC G | 53 | 1-53 |

### Example 10: Affinity of truncated aptdkk-5

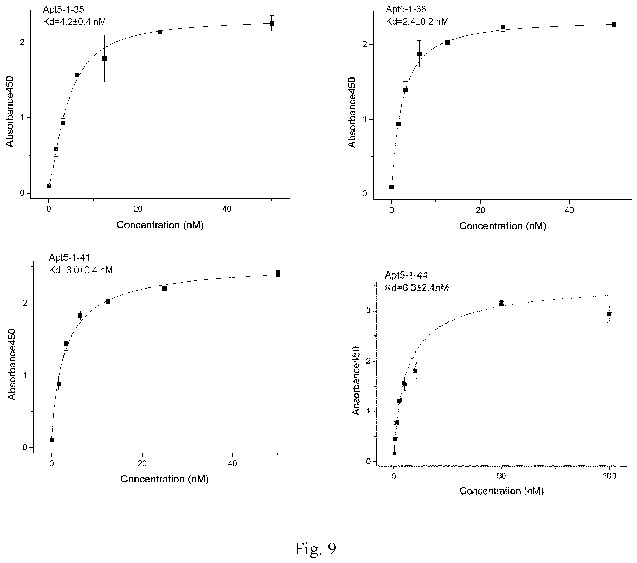

Aptamer candidates with significant specificity (apt5-1-44, apt5-1-41, apt5-1-38, and apt5-1-35) and the original aptdkk5 as a positive control were used to determine the binding affinity against DKK1. The original aptdkk5 and apt5-1-44 had concentrations of (0.15625 nM, 0.625 nM, 1.25 nM, 2.5 nM, 5 nM, 10 nM, 50 nM, and 100 nM), apt5-1-41, apt5-1-38, and apt5-1-35 had concentrations of (0, 1.5625 nM, 3.125 nM, 6.25 nM, 12.5 nM, 25 nM, and 50nM). The binding curve of each candidate was shown in Fig. 9. 11 aptamer candidates showed high affinity (low K_{d} value) against DKK1.

Among these four candidates, apt5-1-38 showed highest affinity against DKK1, so it was selected as the optimal aptamer for further study.

### Example 11: Study on binding sites of truncated Aptdkk5 (Aptdkk5s) with DKK1

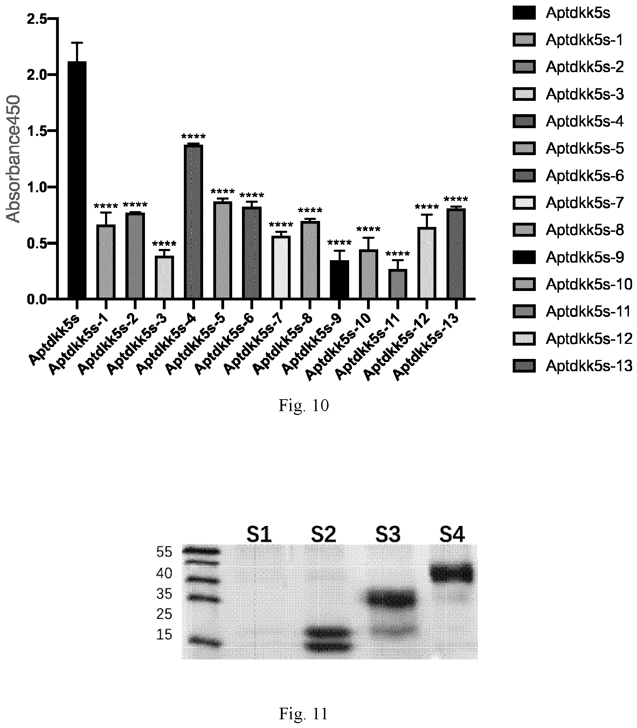

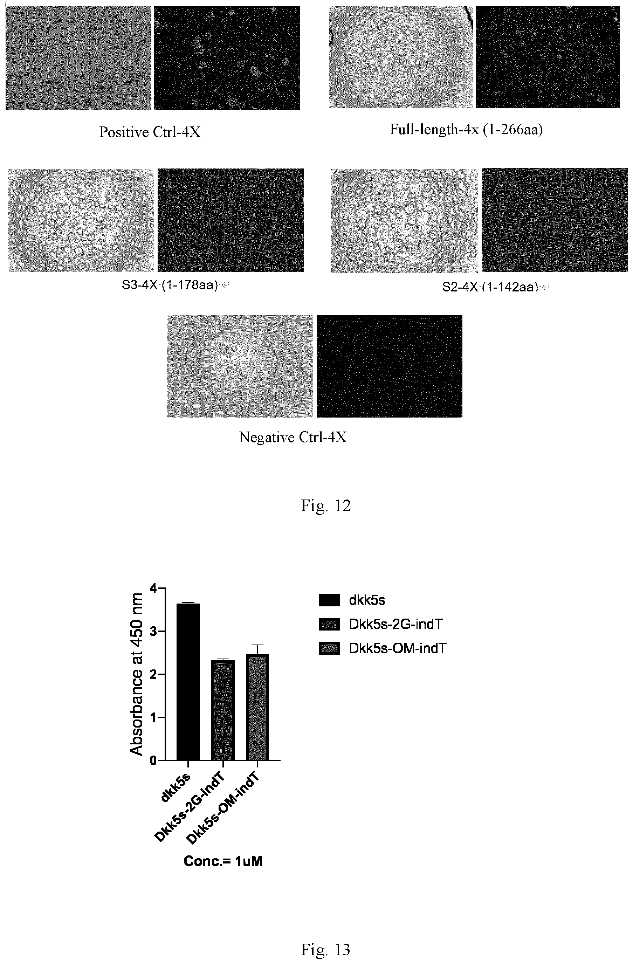

The specificity against DKK-1 was determined with 1 µM of each aptamer/RS for 13 mutated sequences of aptdkk5s and the original aptdkk5s (apt5-1-38 in Example 10) (Table 7) by ELONA, and the absorbance at 450 nm was detected. Compared with the blank control, most of candidate aptamers showed significant lower absorbance, especially aptdkk5s-3, aptdkk5s-9, and aptdkk5s-11 (Fig. 10), indicating that these might be binding sites of aptdkk5s with DKK1. In order to identify a domain of DKK1 binding to aptdkk5s, the wild-type DKK1 plasmid or 3 truncated DKK1 (residues 1-91, 1-142, and 1-178 of SEQ ID NO: 73) plasmids for HEK293T cell transfection were successfully constructed in a vector pCS2 with a His tag. A protein-bead complex was applied for SDS-PAGE gel electrophoresis and the expression of pCS2-DKK 1-His-flag(S4), pCS2-DKK 1-ΔC-His-flag(S3), and pCS2-DKK1-ΔC&L2-His-flag(S2) was verified (Fig. 11). According to the fluorescence image results, it might be seen that aptdkk5s significantly bound to full-length DKK1, while almost did not bind to other truncated DKK1, indicating that the C domain (178-266aa) of DKK1 might be a binding domain of DKK1 with aptdkk5s (Fig. 12).

**Table 7: Sequences of aptdkk5s mutants**

| SEQ ID NO: | Name | Sequence (5' to 3') |
|---|---|---|
| 74 | Aptdkk5s-1 | AAAACGGTCGACGCTAGCTGGTGGTTGGGGTGGGTGGT |
| 75 | Aptdkk5s-2 | CGTAAAGTCGACGCTAGCTGGTGGTTGGGGTGGGTGGT |
| 76 | Aptdkk5s-3 | CGTACGAAAGACGCTAGCTGGTGGTTGGGGTGGGTGGT |
| 77 | Aptdkk5s-4 | CGTACGGTCAAAGCTAGCTGGTGGTTGGGGTGGGTGGT |
| 78 | Aptdkk5s-5 | CGTACGGTCGACAAAAGCTGGTGGTTGGGGTGGGTGGT |
| 79 | Aptdkk5s-6 | CGTACGGTCGACGCTAAATGGTGGTTGGGGTGGGTGGT |
| 80 | Aptdkk5s-7 | CGTACGGTCGACGCTAGCAAATGGTTGGGGTGGGTGGT |
| 81 | Aptdkk5s-8 | CGTACGGTCGACGCTAGCTGGAAATTGGGGTGGGTGGT |
| 82 | Aptdkk5s-9 | CGTACGGTCGACGCTAGCTGGTGGAAAGGGTGGGTGGT |
| 83 | Aptdkk5s-1 0 | CGTACGGTCGACGCTAGCTGGTGGTTGAAATGGGTGGT |
| 84 | Aptdkk5s-11 | CGTACGGTCGACGCTAGCTGGTGGTTGGGGAAAGTGGT |
| 85 | Aptdkk5s-1 2 | CGTACGGTCGACGCTAGCTGGTGGTTGGGGTGGAAAGT |
| 86 | Aptdkk5s-1 3 | CGTACGGTCGACGCTAGCTGGTGGTTGGGGTGGGTGAA |

### Example 12: Specificity and affinity of chemically modified aptdkk5s

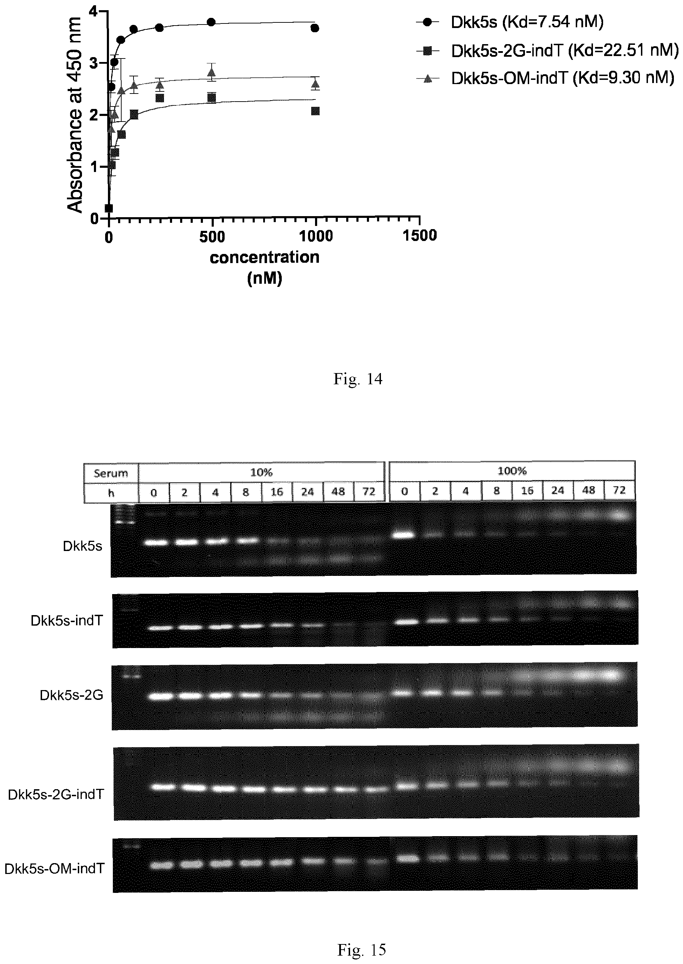

The IDT modification of aptdkk5s was applied for the following characterizations, including dkk5s-2G-indT and dkk5s-OM-indT. Both the modified and original aptdkk5s showed significant high absorbance (>2), while the absorbance of all modified aptdkk5s was slightly lower than that of the original aptdkk5s (Fig. 13), indicating that the specificity of the modified aptdkk5s was very high. Subsequently, the modified aptamer and the original aptdkk5s as a positive control were used to determine the binding affinity against DKK1 with different concentrations (0, 15.625 nM, 31.25 nM, 62.5 nM, 125 nM, 250 nM, 500 nM, and 1000 nM) by ELONA. Both of the modified aptdkk5s (dkk5s-2G-indT and dkk5s-OM-indT) showed high affinity against DKK1, but slightly lower (higher K_{d} value) than the original aptdkk5s (Fig. 14).

### Example 13: Serum stability of chemically modified aptdkk5s

The serum metabolic stability of modified and unmodified aptamers was evaluated in FBS. The aptamers were treated with 10% and 100% FBS for 0 to 72 hours (Fig. 15). The original aptdkk5s began to degrade after being incubated in 10% FBS for 16 hours, and began to degrade from 2 hours in 100% FBS. Dkk5s-2G-indT began to degrade after being incubated in 10% FBS for 72 hours, and began to degrade from 8 hours in 100% FBS. Dkk5s-OM-indT began to degrade after being incubated in 10% FBS for 48 hours, and began to degrade from 8 hours in 100% FBS. In short, compared with the unmodified aptdkk5s, the serum stability of the modified aptdkk5s was significantly improved. In other words, the 3' inverted deoxythymidine of IDT and O-methyl modification both enhanced the serum metabolic stability.

### Example 14: Inhibition efficacy of chemically modified aptdkk5s

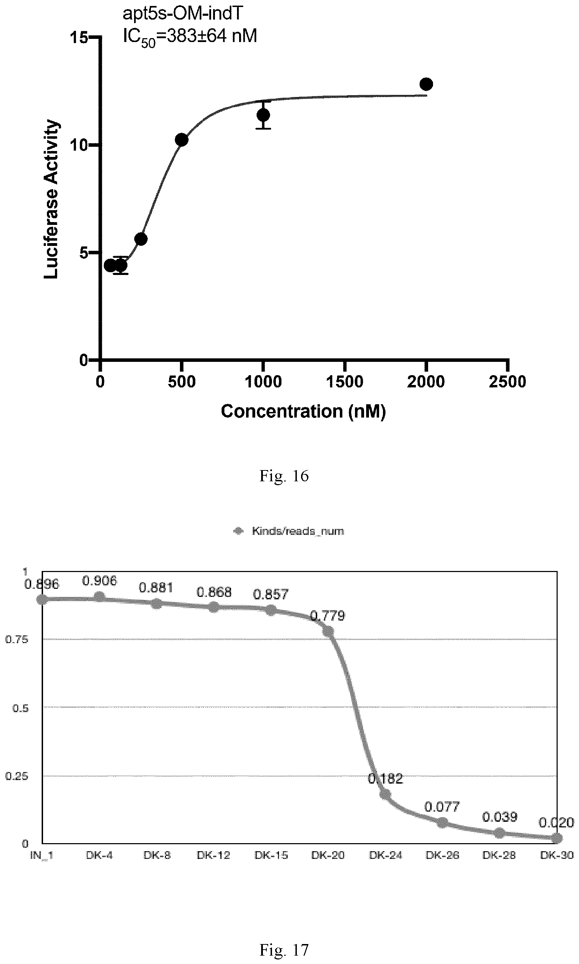

According to the cellular assay results, the inhibition efficacy of the modified aptdkk-5 (aptdkk5-OM-indT) was IC₅₀=383 ± 64 nM, while the inhibition efficacy of the original aptdkk-5 was IC₅₀=754 ± 29 nM, as described above (Fig. 16). The results indicated that the modified aptdkk-5 had higher inhibition effect on the inhibition of Wnt signaling by DKK1.

### Example 15: Further SELEX of aptamer against full-length DKK1

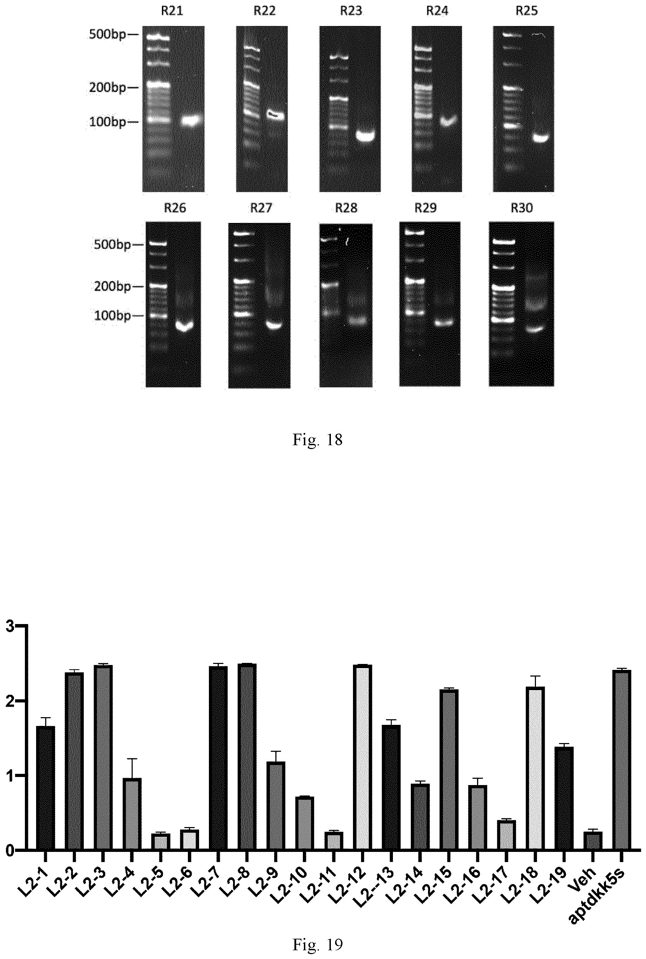

According to data analysis of the last twenty rounds of SELEX against DKK1, the sequence enrichment of the twenty rounds of SELEX was insufficient. Therefore, additional 10 rounds of SELEX against DKK1 were performed under more stringent conditions, to obtain an ssDNA pool with a better enrichment (Figs. 17 and 18). Subsequently, 19 potential sequences were identified in the ssDNA library enriched in the 30th round and named D30-1 to D30-19 (Table 8).

**Table 8: Sequences with highest enrichment in the 30th round**

| SEQ ID NO: | Name | Sequence |
|---|---|---|
| 87 | D30-1 | ATGTCCATTTGCGAGTAGGTGTTTGAGTGAGGTGA |
| 88 | D30-2 | CATTGTCTCAGCGCATGTGTTCAGTTCTTGTGTGT |
| 89 | D30-3 | CTTCTTGTCCCTCGATGTGTTCAGTGTTTGTGTGT |
| 90 | D30-4 | TGCTGAACTTTACGTGATTCTAGTCGCTGACCGCG |
| 91 | D30-5 | ATAGGTTGGGTAGGTTGGTTGCTTTTTTTGTCGCG |
| 92 | D30-6 | TCAGTGTCGTAAAGTGTGTCCCTTTCTTTACGCGC |
| 93 | D30-7 | TGTAGATAGAGGGGTTGGTGTGGTTGGCGCTATCC |
| 94 | D30-8 | TGCATCTTCCTTCTTCGATTCTATGCTCGGCCGCG |
| 95 | D30-9 | CTGTTCTAACGTGGTTGACCCCCCTTTGCTTTGAC |
| 96 | D30-10 | TCCTCTTCCTTTGGCTAGGTTGGTTAGGTTGGTGC |
| 97 | D30-11 | TGGTGTGGGGGTGGGTGGTGGGTTGTATCGTTTGA |
| 98 | D30-12 | TCGTGCTTGATTCCATGCTTGGCCGCCTTCTCGTG |
| 99 | D30-13 | TATGGTTTCCCTACATGTGTTCAGTGTTTGTGTGT |
| 100 | D30-14 | ATGCCCCTCCTCTGTTGAACTGTTTACCCCTTTGA |
| 101 | D30-15 | AATTTCGATAGTCCATGTGTTCAGTGCCTGTGTGT |
| 102 | D30-16 | ATGTAAATCGATTCCTCTGGCCGCATCGCGCTTTA |
| 103 | D30-17 | CAGTCTTAGTGCTCATGTGTTCAGTGTTTGTGTGT |
| 104 | D30-18 | TCTGCTTTCACGTACTAGGTTGGTTAGGTTGGTGT |
| 105 | D30-19 | TACCTTCTGCTCGATGTGTTCAGTGTGTTGTGTGT |

### Example 16: Specificity and affinity of representative aptamer candidates in the 30th round

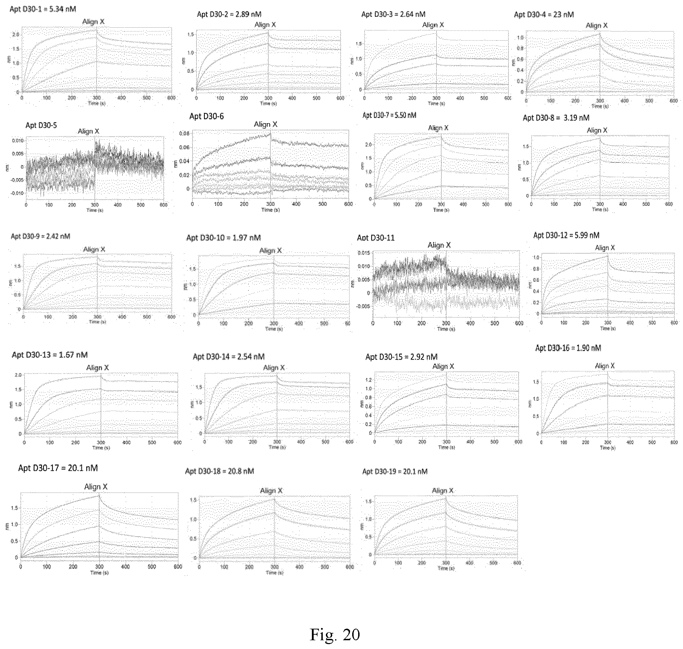

The specificity against DKK-1 was determined with 1 µM of each aptamer/RS for 19 potential sequences with the highest occurrence rate in the 30th round by ELONA. Results indicated that most of the aptamers showed significant higher absorbance compared to the carrier, while Candidates 3, 7, 8, and 12 had higher absorbance compared to the previously identified aptamer aptddk5s (Fig. 19). Next, the binding affinity against DKK1 was determined for candidate aptamers by BLI. DKK1 protein was applied at gradient concentrations of 0 nM, 3.125 nM, 6.25 nM, 12.5 nM, 25 nM, 50 nM, 100 nM, and 200 nM. Except for Candidates 5, 6, and 11 (Table 9, and Fig. 20), 16 candidates having high affinity were detected, and the Kd value was between 1.67 nM and 23 nM.

**Table 9: Summary of specificity and affinity of representative aptamer candidates**

| Aptamer candidate | K_{d} value (nM) | Aptamer candidate | K_{d} value (nM) |
|---|---|---|---|
| D30-1 | 5.34 | D30-11 | / |
| D30-2 | 2.89 | D30-12 | 5.99 |
| D30-3 | 2.64 | D30-13 | 1.67 |
| D30-4 | 23 | D30-14 | 2.54 |
| D30-5 | / | D30-15 | 2.92 |
| D30-6 | / | D30-16 | 1.90 |
| D30-7 | 5.50 | D30-17 | 20.1 |
| D30-8 | 3.19 | D30-18 | 20.8 |
| D30-9 | 2.42 | D30-19 | 20.1 |
| D30-10 | 1.97 | | |

### References:

1. H Kagey M, Xi H. Rationale for targeting the Wnt signalling modulator Dickkopf-1 for oncology. Br J Pharmacol (2017) 174:4637-4650. doi:10.1111/bph.13894
2. D'Amico L, Mahajan S, Capietto AH, Yang Z, Zamani A, Ricci B, Bumpass DB, Meyer M, Su X, Wang-Gillam A, et al. Dickkopf-related protein 1 (Dkk1) regulates the accumulation and function of myeloid derived suppressor cells in cancer. J Exp Med (2016) 213:827-840. doi:10.1084/jem.20150950
3. Malladi S, MacAlinao DG, Jin X, He L, Basnet H, Zou Y, De Stanchina E, Massagué J. Metastatic Latency and Immune Evasion through Autocrine Inhibition of WNT. Cell (2016) 165:45-60. doi:10.1016/j.cell.2016.02.025
4. Arend R, Castro C, Matulonis U, Hamilton E, Gunderson C, LyBarger K, Goodman H, Duska L, Mahdi H, ElNaggar A, et al. 76 Patients (PTS) with recurrent gynecologic cancer whose tumors have activating wnt pathway mutations respond better to DKN-01, a DICKKOPF-1 (DKK1) inhibitor. Int J Gynecol Cancer (2019) 29:A40. doi:10.1136/ijgc-2019-igcs.76
5. Arend RC, Castro CM, Matulonis UA, Hamilton E, Gunderson CC, Lybarger KSS, Kagey M, Sirard C, Birrer MJ. Safety and efficacy of a DKK1 inhibitor (DKN-01) as monotherapy or in combination with paclitaxel in patients with Wnt activated recurrent gynecologic malignancies. Gynecol Oncol (2019) 154:34. doi:10.1016/j.ygyno.2019.04.080
6. Jayasena SD. Aptamers: An emerging class of molecules that rival antibodies in diagnostics. Clin Chem (1999) 45:1628-1650. doi:10.1093/clinchem/45.9.1628
7. Boylan MO, Athanassiou M, Houle B, Wang Y, Zarbl H. Activation of tumor suppressor genes in nontumorigenic revertants of the HeLa cervical carcinoma cell line. Cell Growth Differ (1996)
8. Largy E, Mergny J-L, Gabelica V. "Role of Alkali Metal Ions in G-Quadruplex Nucleic Acid Structure and Stability," in doi:10.1007/978-3-319-21756-7_7
9. Routh ED, Creacy SD, Beerbower PE, Akman SA, Vaughn JP, Smaldino PJ. A G-quadruplex DNA-affinity approach for purification of enzymatically active G4 resolvase1. J Vis Exp (2017) doi:10.3791/55496
10. Sundquist WI, Klug A. Telomeric DNA dimerizes by formation of guanine tetrads between hairpin loops. Nature (1989) doi:10.1038/342825a0

### Sequence List

SEQ ID NO: 1 
CGTACGGTCGACGCTAGCTGTGGTTATGGGGTTGGTGGGTGGGTTCTTACGGA

GGATCCGAGCTCCACGTG
SEQ ID NO: 2 
CGTACGGTCGACGCTAGCTGGTGTTGGGGTGGGTTGGTGGGTATTTTGGCGG

TGGATCCGAGCTCCACGTG
SEQ ID NO: 3 
CGTACGGTCGACGCTAGCTAACTGTCCGACGCGTCTTTCACTTTCTCCCGCCG
GGATCCGAGCTCCACGTG
SEQ ID NO: 4 
CGTACGGTCGACGCTAGCTGCACGCTTTTCCCTCCTTTTCTCGTCTGACCGCG
GGATCCGAGCTCCACGTG
SEQ ID NO: 5 
CGTACGGTCGACGCTAGCTGGTGGTTGGGGTGGGTGGTGGTTGTTCGTGGCC
GGGATCCGAGCTCCACGTG
SEQ ID NO: 6 
CGTACGGTCGACGCTAGCCCGGTTGCAATGGTTGGGGTGGTTGGTTTTGCATG
GGATCCGAGCTCCACGTG
SEQ ID NO: 7 
CGTACGGTCGACGCTAGCAGCCCCCTTCCTTTCTTCCTCGACCTCCCTCCGCA
GGATCCGAGCTCCACGTG
SEQ ID NO: 8 
CGTACGGTCGACGCTAGCTGCCACCCCTGTTTTCCACATTTTCCTCGACCGCG
GGATCCGAGCTCCACGTG
SEQ ID NO: 9 
CGTACGGTCGACGCTAGCTACACCCCTTCCTGTTCTATTCTTCCTGCCTTGCGG
GATCCGAGCTCCACGTG
SEQ ID NO: 10 
CGTACGGTCGACGCTAGCACTCTCCACGTTTTTCCACCTCCTTCCTCCTTTGC
GGATCCGAGCTCCACGTG
SEQ ID NO: 11 
CGTACGGTCGACGCTAGCCATCCAGCTCCCTTTATTCCCTCTGTACCCTCCTGG
GATCCGAGCTCCACGTG
SEQ ID NO: 12 CGTACGGTCGACGCTAGCTCCCCTCATAATCCGACTTTTTCCCTCCCCCTCCCG GATCCGAGCTCCACGTG
SEQ ID NO: 13 
CGTACGGTCGACGCTAGCTGGTGTGGGGGTGGGTGGTGGGTTGTATCGTTTG
AGGATCCGAGCTCCACGTG
SEQ ID NO: 14 
CGTACGGTCGACGCTAGCCTGTGCAGTTGGTTGGTTATGGTTGGGACTGTCAG
GATCCGAGCTCCACGTG
SEQ ID NO: 15 
CGTACGGTCGACGCTAGCACGTTCAGATACCTTTTTTCCCCTTTGTTCTGCCG
GGATCCGAGCTCCACGTG
SEQ ID NO: 16 
CGTACGGTCGACGCTAGCTACACGCCATCGGTGCCCTTTTCTCTTCTTGTCTG
GGATCCGAGCTCCACGTG
SEQ ID NO: 17 
CGTACGGTCGACGCTAGCTACTTCTCCACGTCTCTTTCCCTGTCCCCTTCGCA
GGATCCGAGCTCCACGTG
SEQ ID NO: 18 
CGTACGGTCGACGCTAGCTCCACCCAATTCTCTTCCCGTCTTTCCTGCACATG
GGATCCGAGCTCCACGTG
SEQ ID NO: 19 
CGTACGGTCGACGCTAGCATAGGTTGGGTAGGTTGGTTGCTTTTTTTGTCGCG
GGATCCGAGCTCCACGTG
SEQ ID NO: 20 
CGTACGGTCGACGCTAGCCGTAGGGAGGTGGGTGGGTATTTCTCTCTGTCGTG
GGATCCGAGCTCCACGTG
SEQ ID NO: 21 Apt5-4-71 
ACGGTCGACGCTAGCTGGTGGTTGGGGTGGGTGGTGGTTGTTCGTGGCCGCG
TACGGTCGACGCTACG
SEQ ID NO: 22 Apt5-7-71 
GTCGACGCTAGCTGGTGGTTGGGGTGGGTGGTGGTTGTTCGTGGCCGCGTAC
GGTCGACGCTACG
SEQ ID NO: 23 Apt5-10-71 
GACGCTAGCTGGTGGTTGGGGTGGGTGGTGGTTGTTCGTGGCCGCGTACGGT
CGACGCTACG
SEQ ID NO: 24 Apt5-13-71 
GCTAGCTGGTGGTTGGGGTGGGTGGTGGTTGTTCGTGGCCGCGTACGGTCGA
CGCTACG
SEQ ID NO: 25 Apt5-16-71 
AGCTGGTGGTTGGGGTGGGTGGTGGTTGTTCGTGGCCGCGTACGGTCGACGC
TACG
SEQ ID NO: 26 Apt5-19-71 
TGGTGGTTGGGGTGGGTGGTGGTTGTTCGTGGCCGCGTACGGTCGACGCTAC
G
SEQ ID NO: 27 Apt5-19-53
   TGGTGGTTGGGGTGGGTGGTGGTTGTTCGTGGCCG
SEQ ID NO: 28 Apt5-22-71
   TGGTTGGGGTGGGTGGTGGTTGTTCGTGGCCGCGTACGGTCGACGCTACG
SEQ ID NO: 29 Apt5-25-71
   TTGGGGTGGGTGGTGGTTGTTCGTGGCCGCGTACGGTCGACGCTACG
SEQ ID NO: 30 Apt5-28-71
   GGGTGGGTGGTGGTTGTTCGTGGCCGCGTACGGTCGACGCTACG
SEQ ID NO: 31 Apt5-31-71
   TGGGTGGTGGTTGTTCGTGGCCGCGTACGGTCGACGCTACG
SEQ ID NO: 32 Apt5-34-71
   GTGGTGGTTGTTCGTGGCCGCGTACGGTCGACGCTACG
SEQ ID NO: 33 Apt5-1-50
   CGTACGGTCGACGCTAGCTGGTGGTTGGGGTGGGTGGTGGTTGTTCGTGG
SEQ ID NO: 34 Apt5-1-47
   CGTACGGTCGACGCTAGCTGGTGGTTGGGGTGGGTGGTGGTTGTTCG
SEQ ID NO: 35 Apt5-1-44
   CGTACGGTCGACGCTAGCTGGTGGTTGGGGTGGGTGGTGGTTGT
SEQ ID NO: 36 Apt5-1-41
   CGTACGGTCGACGCTAGCTGGTGGTTGGGGTGGGTGGTGGT
SEQ ID NO: 37 Apt5-1-38
   CGTACGGTCGACGCTAGCTGGTGGTTGGGGTGGGTGGT
SEQ ID NO: 38 Apt5-1-35
   CGTACGGTCGACGCTAGCTGGTGGTTGGGGTGGGT
SEQ ID NO: 39 Apt5-1-68 
CGTACGGTCGACGCTAGCTGGTGGTTGGGGTGGGTGGTGGTTGTTCGTGGCC
GCGTACGGTCGACGCT
SEQ ID NO: 40 Apt5-1-65 
CGTACGGTCGACGCTAGCTGGTGGTTGGGGTGGGTGGTGGTTGTTCGTGGCC
GCGTACGGTCGAC
SEQ ID NO: 41 Apt5-1-62 
CGTACGGTCGACGCTAGCTGGTGGTTGGGGTGGGTGGTGGTTGTTCGTGGCC
GCGTACGGTC
SEQ ID NO: 42 Apt5-1-59 
CGTACGGTCGACGCTAGCTGGTGGTTGGGGTGGGTGGTGGTTGTTCGTGGCC
GCGTACG
SEQ ID NO: 43 Apt5-1-56 
CGTACGGTCGACGCTAGCTGGTGGTTGGGGTGGGTGGTGGTTGTTCGTGGCC
GCGT
SEQ ID NO: 44 Apt5-1-53 CGTACGGTCGACGCTAGCTGGTGGTTGGGGTGGGTGGTGGTTGTTCGTGGCC G
SEQ ID NO: 45
   TGTGGTTATGGGGTTGGTGGGTGGGTTCTTACGGA
SEQ ID NO: 46
   TGGTGTTGGGGTGGGTTGGTGGGTATTTTGGCGGT
SEQ ID NO: 47
   TAACTGTCCGACGCGTCTTTCACTTTCTCCCGCCG
SEQ ID NO: 48
   TGCACGCTTTTCCCTCCTTTTCTCGTCTGACCGCG
SEQ ID NO: 49
   TGGTGGTTGGGGTGGGTGGTGGTTGTTCGTGGCCG
SEQ ID NO: 50
   CCGGTTGCAATGGTTGGGGTGGTTGGTTTTGCATG
SEQ ID NO: 51
   AGCCCCCTTCCTTTCTTCCTCGACCTCCCTCCGCA
SEQ ID NO: 52
   TGCCACCCCTGTTTTCCACATTTTCCTCGACCGCG
SEQ ID NO: 53
   TACACCCCTTCCTGTTCTATTCTTCCTGCCTTGCG
SEQ ID NO: 54
   ACTCTCCACGTTTTTCCACCTCCTTCCTCCTTTGC
SEQ ID NO: 55
   CATCCAGCTCCCTTTATTCCCTCTGTACCCTCCTG
SEQ ID NO: 56
   TCCCCTCATAATCCGACTTTTTCCCTCCCCCTCCC
SEQ ID NO: 57
   TGGTGTGGGGGTGGGTGGTGGGTTGTATCGTTTGA
SEQ ID NO: 58
   CTGTGCAGTTGGTTGGTTATGGTTGGGACTGTCA
SEQ ID NO: 59
   ACGTTCAGATACCTTTTTTCCCCTTTGTTCTGCCG
SEQ ID NO: 60
   TACACGCCATCGGTGCCCTTTTCTCTTCTTGTCTG
SEQ ID NO: 61
   TACTTCTCCACGTCTCTTTCCCTGTCCCCTTCGCA
SEQ ID NO: 62
   TCCACCCAATTCTCTTCCCGTCTTTCCTGCACATG
SEQ ID NO: 63
   ATAGGTTGGGTAGGTTGGTTGCTTTTTTTGTCGCG
SEQ ID NO: 64
   CGTAGGGAGGTGGGTGGGTATTTCTCTCTGTCGTG
SEQ ID NO: 65 QGRS-6
   GGTTGGGGTGGTTGG
SEQ ID NO: 66 QGRS-20
   GGGAGGTGGGTGGG
SEQ ID NO: 67 QGRS-19
   GGTTGGGTAGGTTGG
SEQ ID NO: 68 QGRS-14
   GGTTGGGTAGGTTGG
SEQ ID NO: 69 QGRS-13
   GGGTGGGTGGTGGG
SEQ ID NO: 70 QGRS-5
   GGTTGGGGTGGGTGGTGG
SEQ ID NO: 71 QGRS-1
   GGTTGGTGGGTGGG
SEQ ID NO: 72 QGRS-2
   GGTGGGTTGGTGGG
SEQ ID NO: 73 human DKK1 amino acid sequence 
MMALGAAGATRVFVAMVAAALGGHPLLGVSATLNSVLNSNAIKNLPPPLGGAA
GHPGSAVSAAPGILYPGGNKYQTIDNYQPYPCAEDEECGTDEYCASPTRGGDAG
VQICLACRKRRKRCMRHAMCCPGNYCKNGICVSSDQNHFRGEIEETITESFGND
HSTLDGYSRRTTLSSKMYHTKGQEGSVCLRSSDCASGLCCARHFWSKICKPVLK
EGQVCTKHRRKGSHGLEIFQRCYCGEGLSCRIQKDHHQASNSSRLHTCQRH
SEQ ID NO:74 Aptdkk5s-1
   AAAACGGTCGACGCTAGCTGGTGGTTGGGGTGGGTGGT
SEQ ID NO:75 Aptdkk5s-2
   CGTAAAGTCGACGCTAGCTGGTGGTTGGGGTGGGTGGT
SEQ ID NO:76 Aptdkk5s-3
   CGTACGAAAGACGCTAGCTGGTGGTTGGGGTGGGTGGT
SEQ ID NO:77 Aptdkk5s-4
   CGTACGGTCAAAGCTAGCTGGTGGTTGGGGTGGGTGGT
SEQ ID NO:78 Aptdkk5s-5
   CGTACGGTCGACAAAAGCTGGTGGTTGGGGTGGGTGGT
SEQ ID NO:79 Aptdkk5s-6
   CGTACGGTCGACGCTAAATGGTGGTTGGGGTGGGTGGT
SEQ ID NO:80 Aptdkk5s-7
   CGTACGGTCGACGCTAGCAAATGGTTGGGGTGGGTGGT
SEQ ID NO:81 Aptdkk5s-8
   CGTACGGTCGACGCTAGCTGGAAATTGGGGTGGGTGGT
SEQ ID NO:82 Aptdkk5s-9
   CGTACGGTCGACGCTAGCTGGTGGAAAGGGTGGGTGGT
SEQID NO:83 Aptdkk5s-10
   CGTACGGTCGACGCTAGCTGGTGGTTGAAATGGGTGGT
SEQID NO:84 Aptdkk5s-11
   CGTACGGTCGACGCTAGCTGGTGGTTGGGGAAAGTGGT
SEQ ID NO:85 Aptdkk5s-12
   CGTACGGTCGACGCTAGCTGGTGGTTGGGGTGGAAAGT
SEQID NO:86 Aptdkk5s-13
   CGTACGGTCGACGCTAGCTGGTGGTTGGGGTGGGTGAA
SEQ ID NO:87 D30-1
   ATGTCCATTTGCGAGTAGGTGTTTGAGTGAGGTGA
SEQ ID NO:88 D30-2
   CATTGTCTCAGCGCATGTGTTCAGTTCTTGTGTGT
SEQ ID NO:89 D30-3
   CTTCTTGTCCCTCGATGTGTTCAGTGTTTGTGTGT
SEQ ID NO:90 D30-4
   TGCTGAACTTTACGTGATTCTAGTCGCTGACCGCG
SEQ ID NO:91 D30-5
   ATAGGTTGGGTAGGTTGGTTGCTTTTTTTGTCGCG
SEQ ID NO:92 D30-6
   TCAGTGTCGTAAAGTGTGTCCCTTTCTTTACGCGC
SEQ ID NO:93 D30-7
   TGTAGATAGAGGGGTTGGTGTGGTTGGCGCTATCC
SEQ ID NO:94 D30-8
   TGCATCTTCCTTCTTCGATTCTATGCTCGGCCGCG
SEQ ID NO:95 D30-9
   CTGTTCTAACGTGGTTGACCCCCCTTTGCTTTGAC
SEQ ID NO:96 D30-10
   TCCTCTTCCTTTGGCTAGGTTGGTTAGGTTGGTGC
SEQ ID NO:97 D30-11
   TGGTGTGGGGGTGGGTGGTGGGTTGTATCGTTTGA
SEQ ID NO:98 D30-12
   TCGTGCTTGATTCCATGCTTGGCCGCCTTCTCGTG
SEQ ID NO:99 D30-13
   TATGGTTTCCCTACATGTGTTCAGTGTTTGTGTGT
SEQ ID NO:100 D30-14
   ATGCCCCTCCTCTGTTGAACTGTTTACCCCTTTGA
SEQ ID NO:101 D30-15
   AATTTCGATAGTCCATGTGTTCAGTGCCTGTGTGT
SEQ ID NO:102 D30-16
   ATGTAAATCGATTCCTCTGGCCGCATCGCGCTTTA
SEQ ID NO:103 D30-17
   CAGTCTTAGTGCTCATGTGTTCAGTGTTTGTGTGT
SEQ ID NO:104 D30-18
   TCTGCTTTCACGTACTAGGTTGGTTAGGTTGGTGT
SEQ ID NO:105 D30-19
   TACCTTCTGCTCGATGTGTTCAGTGTGTTGTGTGT

## Claims

1. An aptamer against DKK1, wherein the aptamer
i) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 1-20; or
ii) comprises at least 10, at least 15, at least 20, at least 25, at least 30, at least 35, at least 40, at least 45, at least 50 or more contiguous nucleotides within any one of SEQ ID NOs: 1-20; or
iii) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 45-64; or
iv) comprises a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 87-105,
wherein the aptamer specifically binds to DKK1.

2. The aptamer of claim 1, wherein the aptamer comprises a sequence of any one of SEQ ID NOs: 1-64 and 87-105.

3. The aptamer of claim 1, wherein the aptamer comprises a nucleotide sequence shown in SEQ ID NO: 5, 20, 35-38, 49, or 64, preferably a nucleotide sequence shown in SEQ ID NO: 37.

4. The aptamer of any one of claims 1-3, wherein the aptamer has a K_{d} to DKK1 of less than 350 nM, preferably less than 150 nM, preferably less than 100 nM, preferably less than 50 nM, preferably less than 30 nM, preferably less than 20 nM, preferably less than 10 nM, preferably less than 5 nM, preferably less than 1 nM or less.

5. The aptamer of any one of claims 1-4, wherein the aptamer is capable of inhibiting the biological activity of DKK1.

6. The aptamer of any one of claims 1-5, wherein the aptamer is capable of blocking an antagonistic effect of DKK1 in cell-based Wnt signaling assay.

7. The aptamer of any one of claims 1-6, wherein the aptamer has an IC50 value of less than 2000nM, preferably less than 1500nM, preferably less than 1000nM, preferably less than 800nM or less for inhibiting the biological activity of DKK1, for example, inhibiting the antagonistic effect of DKK1 on Wnt signaling pathway.

8. The aptamer of any one of claims 1-7, wherein the aptamer comprises one or more modifications that confer enhanced nuclease resistance to the aptamer and/or enhance the *in vivo* half-life of the aptamer.

9. The aptamer of any one of claims 1-8, wherein the aptamer comprises 2'-methoxy (2'-OMe) modification and 3' inverted deoxythymidine (3' idT) modification.

10. A method for treating a DKK1-related disease, the method comprises administering a therapeutic effective amount of the aptamer against DKK1 of any one of claims 1-9 to a subject in need thereof, and the subject is, for example, a human.

11. The method of claim 10, wherein the DKK1-related disease is a DKK1-related cancer, such as myeloma (e.g., multiple myeloma with osteolytic lesions, hilar cholangiocarcinoma multiple myeloma), breast cancer, colon cancer, melanoma, hepatocellular cancer, epithelial cancer, esophageal cancer, gastric cancer, gastroesophageal cancer, hilar cholangiocarcinoma, brain cancer, lung cancer, prostate cancer, or pancreatic cancer, as well as any metastases thereof.

12. The method of claim 10, wherein the DKK1-related disease is selected from osteoporosis, osteopenia, osteomalacia, osteogenesis imperfecta (OI), ischemic osteonecrosis, rheumatoid arthritis, fracture, osteoarthritis, and myeloma.

13. A pharmaceutical composition comprising at least one aptamer against DKK1 of any one of claims 1-9 and a pharmaceutically acceptable carrier or excipient.

14. Use of the aptamer against DKK1 of any one of claims 1-9 or the pharmaceutical composition of claim 13 in preparation of a medicament for treating a DKK1-related disease.

15. The use of claim 14, wherein the DKK1-related disease is a DKK1-related cancer, such as myeloma (e.g., multiple myeloma with osteolytic lesions, hilar cholangiocarcinoma multiple myeloma), breast cancer, colon cancer, melanoma, hepatocellular cancer, epithelial cancer, esophageal cancer, gastric cancer, gastroesophageal cancer, hilar cholangiocarcinoma, brain cancer, lung cancer, prostate cancer, or pancreatic cancer, as well as any metastases thereof.

16. The use of claim 14, wherein the DKK1-related disease is selected from osteoporosis, osteopenia, osteomalacia, osteogenesis imperfecta (OI), ischemic osteonecrosis, rheumatoid arthritis, fracture, osteoarthritis, and myeloma.
